# EUROPÄISCHE PATENTSCHRIFT

(11) **EP 1 237 859 B1**
(45) Veröffentlichungstag und Bekanntmachung des Hinweises auf die Patenterteilung: **12.12.2007**
(21) Anmeldenummer: 00962330.7
(22) Anmeldetag: 17.08.2000
(51) Int. Cl.: C07C 315/00

(54) **VERWENDUNG VON BISSULFONAMIDEN ZUR HERSTELLUNG VON MEDIKAMENTEN ZUR PROPHYLAXE ODER BEHANDLUNG VON HYPERLIPIDÄMIE**
USE OF BIS-SULFONAMIDES FOR PRODUCING MEDICAMENTS USED FOR PREVENTING OR TREATING HYPERLIPIDAEMIA
UTILISATION DE BISULFONAMIDES POUR PREPARER DES MEDICAMENTS S'UTILISANT POUR ASSURER LA PROPHYLAXIE OU LE TRAITEMENT DE L'HYPERLIPIDEMIE

(30) Priorität: 01.09.1999 DE 19941559
(43) Veröffentlichungstag der Anmeldung: 11.09.2002
(73) Patentinhaber: Sanofi-Aventis Deutschland GmbH, 65929 Frankfurt am Main (DE)
(72) Erfinder: KIRSCH, Reinhard, 38100 Braunschweig (DE); SCHAEFER, Hans-Ludwig, 65239 Hochheim (DE); FALK, Eugen, 60529 Frankfurt (DE); KRASS, Norbert, 65719 Hofheim (DE)
(86) Internationale Anmeldenummer: PCT/EP2000/008026
(87) Internationale Veröffentlichungsnummer: WO 2001/016096

(56) Entgegenhaltungen:
- EP-A- 0 081 425
- EP-A- 0 288 028
- EP-A- 0 384 279

## Beschreibung

Die Erfindung betrifft die Verwendung Bissulfonamiden sowie derer physiologisch verträgliche Salze und physiologisch funktionellen Derivate zur Herstellung von Medikamenten zur Prävention und Behandlung von Hyperlipidämie sowie arteriosklerotischer Erkrankungen.

In US 3,876,632 sind Bissulfonamide als Antihypertonika beschrieben.

Der Erfindung lag die Aufgabe zugrunde, Verbindungen zur Verfügung zu stellen, die eine therapeutisch verwertbare hypolipidämische Wirkung entfalten.

Die Erfindung betrifft daher die Verwendung der Verbindungen der Formel I, 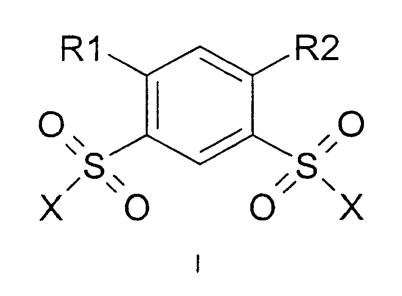 worin bedeuten
- X, R1, R2: unabhängig voneinander NR6R7, Pyrrolidin, Piperidin, Piperazin, Morpholin, Tetrahydropyridin, wobei die Ringe jeweils substituiert sein können mit Phenyl, (C₁-C₆)-Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl, wobei der Phenyl-Substituent unsubstituiert oder bis zu zweifach substituiert ist mit F, Cl, Br, OH, CF₃, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, CONH(C₃-C₆)Cycloalkyl, NH₂, NH-CO-(C₁-C₆)-Alkyl, NH-CO-Phenyl;
- R6 und R7: unabhängig voneinander H, (C₁-C₆)-Alkyl, (C₁-C₆)-Alkyl-O-(C₁-C₆)-Alkyl,O-(C₁-C₆)-Alkyl ,(C₃-C₆)-Cycloalkyl, CO-(C₁-C₆)-Alkyl, (C₁-C₆)-Alkyl-NH-C(O)-(C₁-C₆)-Alkyl, (C₁-C₆)-Alkyl-NH-(C₁-C₆)-Alkyl, (C₁-C₆)-Alkyl-N-[(C₁-C₆)-Alkyl]₂, (C₁-C₆)-Alkyl-O-Phenyl, CHO, CO-Phenyl,
(CH₂)ₙ-Ar, wobei n = 0 - 6 sein kann und Ar gleich Phenyl, Biphenylyl, 1- oder 2-Naphthyl, 1- oder 2-Tetrahydrofuranyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3-, 4- oder 5-Isoxazolyl, (C₃-C₆)-Cycloalkyl, Piperidinyl, Pyrrolidinyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2-, 3- oder 4-Morpholinyl, 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und Ar zu zweifach mit F, Cl, Br, OH, CF₃, NO₂, CN, OCF₃, O-CH₂-O, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, CONH(C₃-C₆)Cycloalkyl, NH₂, NH-CO-(C₁-C₆)-Alkyl, NH-CO-Phenyl, Pyrrolidin-1-yl, Morpholin-1-yl, Piperidin-1-yl, Piperazin-1-yl, 4-Methyl-piperazin-1-yl, (CH₂)ₙ-Phenyl, O-(CH₂)ₙ-Phenyl, S-(CH₂)ₙ-Phenyl, SO₂-(CH₂)ₙ-Phenyl, wobei n = 0-3, substituiert sein kann;
sowie derer physiologisch verträgliche Salze und physiologisch funktionellen Derivate zur Herstellung eines Medikamentes zur Prävention und Behandlung von Hyperlipidämie.

Bevorzugt ist die Verwendung der Verbindungen der Formel I, in denen ein oder mehrere Rest(e) die folgende Bedeutung hat bzw. haben:
- R1: NR6R7, Pyrrolidin, Piperidin, Piperazin, Tetrahydropyridin, wobei die Ringe jeweils substituiert sein können mit Phenyl, (C₁-C₆)-Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl, wobei der Phenyl-Substituent unsubstituiert oder bis zu zweifach substituiert ist mit F, Cl, Br, CF₃, CN, OCF₃, O-(C₁-C₆)-Alkyl, S-(C₁-C₆)-Alkyl, (C₁-C₆)-Alkyl, (C₃-C₆)-Cycloalkyl, COOH, COO(C₁-C₆)-Alkyl, COO(C₃-C₆)Cycloalkyl, CONH₂, CONH(C₁-C₆)Alkyl, CON[(C₁-C₆)Alkyl]₂, NH₂, NH-CO-(C₁-C₆)-Alkyl, NH-CO-Phenyl;
- R6, R7: unabhängig voneinander H, (C₁-C₆)-Alkyl, (C₁-C₆)-Alkyl-O-(C₁-C₆)-Alkyl, (C₃-C₆)-Cycloalkyl, CO-(C₁-C₆)-Alkyl, (C₁-C₆)-Alkyl-NH-C(O)-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, Biphenylyl, 1- oder 2-Naphthyl, 2-, 3- oder 4-Pyridyl, 2- oder 3-Thienyl, 2-, 4- oder 5-Thiazolyl, 2-, 4- oder 5-Oxazolyl, 3- oder 5-Isoxazolyl, (C₃-C₆)-Cycloalkyl, Piperidinyl, Pyrrolidinyl, 2-, 4- oder 5-Pyrimidinyl, 2-, 3- oder 4-Morpholinyl, 2- oder 5-Benzimidazolyl, 2-Benzothiazolyl oder Indol-3-yl, Indol-5-yl sein kann und Ar bis zu zweifach substituiert sein kann mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, NH₂, NH-CO-Phenyl, (CH₂)ₙ-Phenyl, O-(CH₂)ₙ-Phenyl, S-(CH₂)ₙ-Phenyl, wobei n = 0-3, substituiert sein kann;
- R2: NR8R9, Piperazin, wobei Piperazin substituiert sein kann mit (C₁-C₆)-Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl;
- R8, R9: unabhängig voneinander H, (C₁-C₆)-Alkyl, (C₃-C₆)-Cycloalkyl, CO-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 2-, 3- oder 4-Pyridyl, Piperidinyl, Pyrrolidinyl, Morpholinyl sein kann;
- X: NR10R11, Pyrrolidin, Piperidin, Piperazin, Morpholin, wobei die Ringe jeweils substituiert sein können mit Phenyl, (C₁-C₆)Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl, wobei der Phenyl-Substituent unsubstituiert oder bis zu zweifach substituiert ist mit F, Cl, Br, CF₃, CN, OCF₃, O-(C₁-C₆)-Alkyl, (C₁-C₆)-Alkyl, (C₃-C₆)-Cycloalkyl, COOH, COO(C₁-C₆)-Alkyl, COO(C₃-C₆)Cycloalkyl, CONH₂, CONH(C₁-C₆)Alkyl, CON[(C₁-C₆)Alkyl]₂, NH₂, NH-CO-(C₁-C₆)-Alkyl, NH-CO-Phenyl;
- R10, R11: unabhängig voneinander H, (C₁-C₆)-Alkyl, , (C₁-C₆)-Alkyl-O-(C₁-C₆)-Alkyl, (C₃-C₆)-Cycloalkyl, CO-(C₁-C₆)-Alkyl, (C₁-C₆)-Alkyl-NH-(C₁-C₆)-Alkyl, (C₁-C₆)-Alkyl-N-[(C₁-C₆)-Alkyl]₂, CO-Phenyl, (CH₂)ₙ-Ar, wobei n = 0 - 6 sein kann und Ar gleich Phenyl, Biphenylyl, 1- oder 2-Naphthyl, 1- oder 2-Tetrahydrofuranyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 3- oder 5-Isoxazolyl, Piperidinyl, Pyrrolidinyl, 2-, 4- oder 5-Pyrimidinyl, 2-, 3- oder 4-Morpholinyl, 2-Benzothiazolyl sein kann und Ar zweifach substituiert sein kann mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, CONH₂, CONH(C₁-C₆)Alkyl, CON[(C₁-C₆)Alkyl]₂, NH-CO-(C₁-C₆)-Alkyl, NH-CO-Phenyl, (CH₂)ₙ-Phenyl, wobei n = 0-3, substituiert sein kann;
sowie derer physiologisch verträgliche Salze und physiologisch funktionellen Derivate zur Herstellung eines Medikamente zur Prävention und Behandlung von Hyperlipidämie.

Besonders bevorzugt ist die Verwendung von Verbindungen der Formel I, in denen ein oder mehrere Rest(e) die folgende Bedeutung hat bzw. haben:
- R1: NR6R7, Piperidin, Piperazin, Tetrahydropyridin, wobei die Ringe jeweils substituiert sein können mit Phenyl, (C₁-C₆)-Alkyl-Phenyl;
- R6, R7: unabhängig voneinander H, (C₁-C₆)-Alkyl, (C₁-C₆)-Alkyl-O-(C₁-C₆)-Alkyl, (C₃-C₆)-Cycloalkyl, (C₁-C₆)-Alkyl-NH-C(O)-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 1- oder 2-Naphthyl, 2-, 3- oder 4-Pyridyl, (C₃-C₆)-Cycloalkyl, Piperidinyl, Pyrrolidinyl, 2-, 4- oder 5-Pyrimidinyl, 2-, 3- oder 4-Morpholinyl sein kann und Ar bis zu zweifach substituiert sein kann mit F, Cl, Br, OH, CF₃, NO₂, CN, OCF₃, O-(C₁-C₆)-Alkyl, (C₁-C₆)-Alkyl, NH₂;
- R2: NR8R9, Piperazin, wobei Piperazin substituiert sein kann mit (C₁-C₆)-Alkyl;
- R8, R9: unabhängig voneinander H, (C₁-C₆)-Alkyl, (C₃-C₆)-Cycloalkyl, CO-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 2-, 3- oder 4-Pyridyl, Piperidinyl, Pyrrolidinyl, Morpholinyl sein kann;
- X: NR10R11, Pyrrolidin, Piperidin, Morpholin, wobei die Ringe jeweils substituiert sein können mit Phenyl, (C₁-C₆)-Alkyl-Phenyl;
- R10, R11: unabhängig voneinander H, (C₁-C₆)-Alkyl, (C₁-C₆)-Alkyl-O-(C₁-C₆)-Alkyl, (C₃-C₆)-Cycloalkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 2- oder 3-Thienyl;
sowie derer physiologisch verträgliche Salze zur Herstellung eines Medikaments zur Prävention und Behandlung von Hyperlipidämie.

Die Erfindung bezieht sich auf Verwendung der Verbindungen der Formel I, in Form ihrer Racemate, racemischen Mischungen und reinen Enantiomere sowie auf ihre Diastereomere und Mischungen davon.

Die Alkyl-, Alkenyl- und Alkinylreste in den Substituenten X, R1 und R2 können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der Verbindungen der Formel I sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isäthion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chloridsalz und das Weinsäuresalz, verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung, z.B. ein Ester, das bei Verabreichung an einen Säuger, wie z.B. den Menschen, in der Lage ist, (direkt oder indirekt) eine solche Verbindung oder einen aktiven Metaboliten hiervon zu bilden.

Ein weiterer Aspekt dieser Erfindung ist die Verwendung von Prodrugs der Verbindungen der Formel I. Solche Prodrugs können in vivo zu einer Verbindung der Formel I metabolisiert werden. Diese Prodrugs können selbst wirksam sein oder nicht.

Die Verbindungen der Formel I können auch in verschiedenen polymorphen Formen vorliegen, z.B. als amorphe und kristalline polymorphe Formen. Alle polymorphen Formen der Verbindungen der Formel I gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des Salzes der Verbindung der Formel (I). Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I).

Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die folgenden Zubereitungen dienen zur Erläuterung der Erfindung ohne diese jedoch einzuschränken.

### Beispiel A

Gelatineweichkapseln, enthaltend 100 mg Wirkstoff pro Kapsel:

| | pro Kapsel |
|---|---|
| Wirkstoff aus Kokosfett fraktioniertes | 100 mg |
| Triclycerid-Gemisch | 400 mg |
| Kapselinhalt | 500 mg |

### Beispiel B

Emulsion, enthaltend 60 mg Wirkstoff pro 5 ml:

| | pro 100 ml Emulsion |
|---|---|
| Wirkstoff | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

### Beispiel C

Rektale Arzneiform, enthaltend 40 mg Wirkstoff pro Suppositorium:

| | pro Suppositorium |
|---|---|
| Wirkstoff | 40 mg |
| Suppositoriengrundmasse | ad 2 g |

### Beispiel D

Tabletten, enthaltend 40 mg Wirkstoff pro Tablette:

| | pro Tablette |
|---|---|
| Lactose | 600 mg |
| Maisstärke | 300 mg |
| lösliche Stärke | 20 mg |
| Magnesiumstearat | 40 mg |
| | 1000 mg |

### Beispiel E

Dragees, enthaltend 50 mg Wirkstoff pro Dragees:

| | pro Dragee |
|---|---|
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

### Beispiel F

Für die Herstellung des Inhalts von Hartgelatinekapseln eignen sich die folgenden Rezepturen:

| | | |
|---|---|---|
| a) | Wirkstoff | 100 mg |
| | Maisstärke | 300 mg |
| | | 400 mg |
| b) | Wirkstoff | 140 mg |
| | Milchzucker | 180 mg |
| | Maisstärke | 180 mg |
| | | 500 mg |

### Beispiel G

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |
| Benzoesäureethylester | 0,03 g |
| Ethanol 96 %ig | 5 ml |
| entimineralisiertes Wasser | ad 100 ml |

Gegenstand der Erfindung ist weiterhin ein Verfahren zur Herstellung der Verbindungen der allgemeinen Formel I, dadurch gekennzeichnet, daß man Verbindungen der allgemeinen Formel I gemäß folgendem Reaktionsschema darstellt:

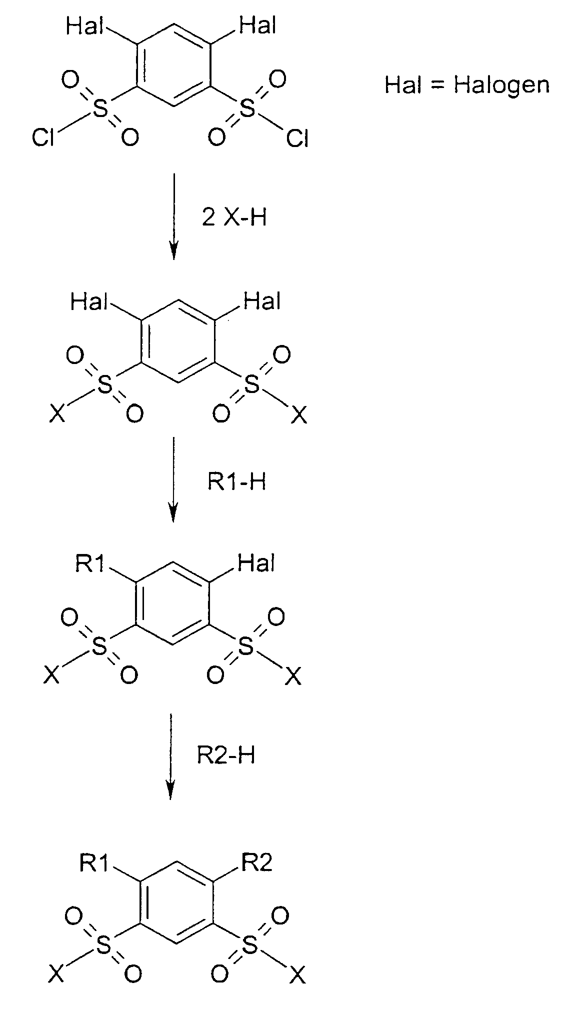

Die nachfolgend aufgeführten Beispiele dienen zur Erläuterung der Erfindung, ohne diese jedoch einzuschränken. Die angegebenen Zersetzungspunkte sind nicht korrigiert und generell von der Aufheizgeschwindigkeit abhängig.

**Tabelle 1: Beispiele**

| | | | | | | | |
|---|---|---|---|---|---|---|---|
| 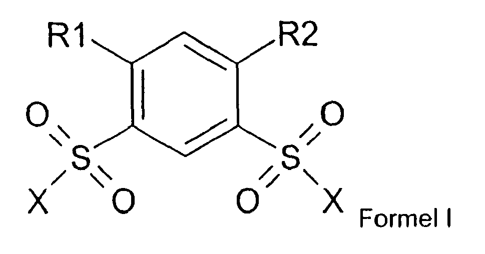 | | | | | | | |

| Bsp. | R1 | R2 | X | Summenformel | MW | MS | Mp (°C) |
|---|---|---|---|---|---|---|---|
| | | | | | | (M+H⁺) | |
| 1 | 4-N(CH₃)-piperazin-1-yl | 4-N(CH₃)-piperazin-1-yl | NH-cyclohexyl | C28 H48 N6 O4 S2 | 596,9 | 597.3 | 232,5 |
| 2 | NH-benzyl | 4-N(CH₃)-piperazin-1-yl | NH-cyclohexyl | C30 H45 N5 O4 S2 | 603,8 | 604,3 | 194 |
| 3 | 4-N(CH₃)-piperazin-1-yl | 4-N(CH₃)-piperazin-1-yl | N(CH)₃-Benzyl | C32 H44 N6 O4 S2 | 640,9 | 641,3 | 124 |
| 4 | NH-benzyl | 4-N(CH₃)-piperazin-1-yl | NH-phenyl | C30 H33 N5 O4 S2 | 591,8 | 592,3 | 211 |
| 5 | NH-benzyl | 4-N(CH₃)-piperazin-1-y) | Pyrrolidin-1-yl | C26 H37 N5 O4 S2 | 547,7 | 548,3 | 173 |
| 6 | 4-N(CH₃)-piperazin-1-yl | 4-N(CH₃)-piperazin-1-yl | NH-phenyl | C30 H40 N6 O4 S2 | 612,8 | 613,3 | 189 |
| 7 | 4-N(CH₃)-piperazin-1-yl | 4-N(CH₃)-piperazin-1-yl | N(CH₃)-phenyl | C30 H40 N6 O4 S2 | 612,8 | 613,3 | 175 |
| 8 | 4-N(CH₃)-piperazin-1-yl | 4-N(CH₃)-piperazin-1-yl | Piperidin-1-yl | C26 H44 N6 O4 S2 | 568,8 | 569,3 | 202,5 |
| 9 | NH-benzyl | 4-N(CH₃)-piperazin-1-yl | N(CH)₃-phenyl | C32 H37 N5 O4 S2 | 619,8 | 620,2 | 149 |
| 10 | NH-CH₂-pyrid-2-yl | 4-N(CH₃)-piperazin-1-yl | N(CH)₃-phenyl | C31 H36 N6 O4 S2 | 620,8 | 621,3 | 155,5 |
| 11 | NH-CH₂-(3,4-methylendioxyphenyl) | 4-N(CH₃)-piperazin-1-y1 | Pyrrolidin-1-yl | C27 H37 N5 O6 S2 | 591,8 | 591,3 | 118 (Zers.) |
| 12 | NH-benzyl | 4-N(CH₃)-piperazin-1-yl | NH-CH₂-CH(CH₃)₂ | C26 H41 N5 O4 S2 | 551,8 | 552,3 | 169 |
| 13 | NH-CH₂-cyclohexyl | 4-N(CH₃)-piperazin-1-yl | Morpholin-4-yl | C26 H43 N5 O6 S2 | 585,8 | 586,3 | 249 |
| 14 | NH-CH₂-tetrahydrofuran-2-yl | 4-N(CH₃)-piperazin-1-yl | N(CH)₃-phenyl | C30 H39 N5 O5 S2 | 613,8 | 614,3 | 147,5 |
| 15 | NH-propyl-phenyl | 4-N(CH₃)-piperazin-1-yl | Morpholin-4-yl | C28 H41 N5 O6 S2 | 607,8 | 608,3 | 167 |
| 16 | NH-benzyl | 4-N(CH₃)-piperazin-1-yl | N(ethyl)₂ | C26 H41 N5 O4 S2 | 551,8 | | 89 |
| 17 | NH-propyl-phenyl | 4-N(CH₃)-piperazin-1-yl | Piperidin-1-yl | C30 H45 N5 O4 | 603,8 | | 134 |
| 18 | NH-propyl-phenyl | 4-N(CH₃)-piperazin-1-yl | NH-benzyl | C34 H41 N5 O4 S2 | 647,9 | | 152 |
| 19 | NH-CH₂-cyclohexyl | 4-N(CH₃)-piperazin-1-yl | N(ethyl)₂ | C26 H47 N5 O4 | 557,8 | | 109 |
| 20 | NH-propyl-phenyl | 4-N(CH₃)-piperazin-1-yl | N(ethyl)₂ | S2C28 H45 N5 O4 S2 | 579,8 | 580,3 | Öl |
| 21 | NH-ethyl-phenyl | 4-N(CH₃)-piperazin-1-yl | Piperidin-1-yl | C29 H43 N5 O4 S2 | 589,8 | 590,3 | 70 |
| 22 | NH-ethyl-phenyl | 4-N(CH₃)-piperazin-1-yl | N(ethyl)₂ | C27 H43 N5 O4 S2 | 565,8 | | Öl |
| 23 | NH-ethyl-phenyl | 4-N(CH₃)-piperazin-1-yl | N(CH)₃-phenyl | C33 H39 N5 O4 S2 | 633,8 | 634,3 | 168 |
| 24 | NH-CH₂-cyclohexyl | 4-N(CH₃)-piperazin-1-yl | N(CH)₃-phenyl | C32 H43 N5 O4 S2 | 625,9 | | 174 |
| 25 | NH-propyl-phenyl | 4-N(CH₃)-piperazin-1-yl | N(CH)₃-phenyl | C34 H41 N5 O4 S2 | 647,9 | | 132 |
| 26 | NH-propyl-phenyl | 4-N(CH₃)-piperazin-1-yl | Pyrrolidin-1-yl | C28 H41 N5 O4 S2 | 575,8 | | 160 |
| 27 | NH-propyl-phenyl | 4-N(CH₃)-piperazin-1-yl | N(CH₃)-benzyl | C36 H45 N5 O4 S2 | 675,9 | | 113 |
| 28 | NH-ethyl-phenyl | 4-N(CH₃)-piperazin-1-yl | N-morpholinyl | C27 H39 N5 O6 S2 | 593,8 | 594,3 | 181 |
| 29 | NH-ethyl-N-(Et)₂ | 4-N(CH₃)-piperazin-1-yl | NH-CH₂-CH(CH₃)₂ | C25 H48 N6 O4 S2 | 560,8 | 561,3 | 158 |
| 30 | NH-CH₂-pyrid-3-yl | NH-ethyl-N(CH₃)₂ | N(CH₃)-benzyl | C32 H40 N6 O4 S2 | 636,8 | 637,3 | |
| 31 | NH-ethyl-N(ethyl)₂ | NH-ethyl-N(CH₃)₂ | N(CH₃)-benzyl | C32 H48 N6 O4 S2 | 644,9 | 645,3 | |
| 32 | NH-propyl-O-CH₃ | NH-ethyl-N(CH₃)₂ | N(CH₃)-benzyl | C30 H43 N5 O5 S2 | 617,8 | 618_{,}3 | |
| 33 | NH-ethyl-N-morpholin-4-yl | NH-ethyl-N-pyrrolidin-1-yl | N(CH₃)-benzyl | C34 H48 N6 O5 S2 | 684,9 | 685,3 | |
| 34 | NH-ethyl-N-pyrrolindin-1-yl | NH-ethyl-NH-acetyl | N(CH₃)-benzyl | C32 H44 N6 O5 S2 | 656,9 | 657,3 | |
| 35 | NH-propyl-O-CH₃ | NH-ethyl-N-pyrrolidin-1-yl | N(CH₃)-benzyl | C32 H45 N5 O5 S2 | 643,9 | 644,3 | |
| 36 | N-piperidinyl | N(ethyl)-ethyl-N(CH₃)₂ | N(CH₃)-benzyl | C33 H47 N5 O4 S2 | 641,9 | 642,3 | |
| 37 | NH-ethyl-N-morpholin-4-yl | NH-ethyl-N-pyrrolidin-1-yl | N(CH)₃-phenyl | C30 H42 N6 O5 S2 | 630,8 | 631,3 | |
| 38 | NH-benzyl | NH-ethyl-N(CH₃)₂ | N(CH)₃₋phenyl | C31 H37 N5 O4 S2 | 607,8 | 608,2 | |
| 39 | NH-ethyl-N(ethyl)₂ | NH-ethyl-N(CH₃)₂ | N(CH)₃-phenyl | C30 H44 N6 O4 S2 | 616,8 | 617,3 | |
| 40 | NH-propyl-O-CH₃ | NH-ethyl-N-pyrrolidin-1-yl | N(CH)₃₋phenyl | C30 H41 N5 O5 S2 | 615,8 | 616,3 | |
| 41 | N-piperidinyl | N(ethyl)-ethyl-N(CH₃)₂ | N(CH)₃-phenyl | C31 H43 N5 O4 S2 | 613,8 | 614,3 | |
| 42 | NH-ethyl-N-pyrrolidin-1-yl | NH-ethyl-N-pyrrolidin-1-yl | N(CH)₃-phenyl | C32 H46 N6 O4 S2 | 642,9 | 643,3 | |
| 43 | NH-benzyl | NH-ethyl-N-pyrrolidin-1-yl | N(CH)₃-phenyl | C33 H39 N5 O4 S2 | 633,8 | 634,3 | |
| 44 | N-piperidinyl | NH-ethyl-N-pyrrolidin-1-yl | N(CH)₃-phenyl | C31 H41 N5 04 S2 | 611,8 | 612,3 | |
| 45 | NH-ethyl-morpholin-4-yl | NH-ethyl-N-pyrrolidin-1-yl | N(CH)₃-phenyl | C32 H44 N6 O5 S2 | 656,9 | 657,3 | |
| 46 | NH-CH₂-pyrid-2-yl | Piperazin-1-yl | N(CH)₃-phenyl | C30 H34 N6 O4 S2 | 606,8 | 607,3 | 89 |
| 47 | NH-CH₂-cyclohexyl | 4-N(CH₃)-piperazin-1-yl | NH-piperidin-4-yl-(1-N-ethyl-phenyl) | C44 H65 N7 O4 S2 | 820,2 | | 175 |
| 48 | NH-benzyl | 4-N(CH₃)-piperazin-1-yl | NH-piperidin-4-yl-(1-N-ethyl-phenyl) | C44 H59 N7 O4 S2 | 814,1 | | 111 (Zers.) |
| 49 | NH-ethyl-phenyl | 4-N(CH₃)-piperazin-1-yl | NH-piperidin-4-yl-(1-N-ethyl-phenyl) | C45 H61 N7 04 S2 | 828,2 | | 84 (Zers.) |
| 50 | NH-propyl-phenyl | 4-N(CH₃)-piperazin-1-yl | NH-piperidin-4-yl-(1-N-ethyl-phenyl) | C46 H63 N7 O4 S2 | 842,2 | | 118 |
| 51 | Piperidin-1-yl | N(ethyl)-ethyl-N(CH₃)₂ | NH-ethyl-N(ethyl)₂ | C29 H57 N7 O4 S2 | 631,9 | 632,4 | |
| 52 | NH-ethyl-phenyl | NH-CH(CH₃)-propyl-N(ethyl)₂ | Piperidin-1-yl | C33 H53 N5 O4 S2 | 647,9 | 648,4 | |
| 53 | NH-ethyl-thien-2-yl | piperazin-1-yl-4-ethyl-OH | Piperidin-1-yl | C28 H43 N5 O5 S3 | 625,9 | 626,3 | |
| 54 | NH-ethyl-thien-2-yl | NH-CH(CH₃)-(CH₂)₃-N(ethyl)₂ | Piperidin-1-yl | C31 H51 N5 O4 S3 | 654,0 | 654,3 | |
| 55 | NH-ethyl-N(CH₃)₂ | NH-ethyl-N(ethyl)₂ | NH-phenyl | C28 H40 N6 O4 S2 | 588,8 | 589,3 | |
| 56 | NH-ethyl-phenyl | NH-ethyl-N(CH₃)₂ | NH-ethyl-N(ethyl)₂ | C30 H53 N7 O4 S2 | 639,9 | 640,4 | |
| 57 | N(ethyl)-ethyl-N(CH₃)₂ | 4-N(CH₃)-piperazin-1-yl | NH-ethyl-thien-2-yl | C29 H44 N6 O4 S4 | 668,2 | 669,2 | |
| 58 | NH-benzyl | N-ethyl-N-pyrrolidin | N(ethyl)-ethyl-N(CH₃)₂ | C31 H53 N7 O4 S2 | 651,9 | 652,3 | |
| 59 | N(CH₃)-benzyl | NH-ethyl-N-pyrrolidin | N(ethyl)-ethyl-N(CH₃)₂ | C32 H55 N7 O4 S2 | 666,0 | 666,3 | |
| 60 | NH-benzyl | 4-N(CH₃)-piperazin-1-yl | N(ethyl)-ethyl-N(CH₃)₂ | C30 H51 N7 O4 S2 | 637,9 | 638,3 | |
| 61 | N(CH₃)-benzyl | 4-N(CH₃)-piperazin-1-yl | N(ethyl)-ethyl-N(CH₃)₂ | C31 H53 N7 O4 S2 | 651,9 | 652,3 | |
| 62 | NH-ethyl-phenyl | NH-ethyl-N-pyrrolidinyl | N(ethyl)-ethyl-N(CH₃)₂ | C32 H55 N7 O4 S2 | 666,0 | 666,3 | |
| 63 | N(CH₃)-benzyl | NH-ethyl-N-pyrrolidinyl | NH-CH₂-cyclopropyl | C28 H41 N5 O4 S2 | 575,8 | 576,3 | |
| 64 | Piperazin-1-yl-4-phenyl | N(ethyl)-ethyl-N(CH₃)₂ | Piperidin-1-yl | C32 H50 N6 O4 S2 | 646,9 | 647,3 | 116 - 118 |
| 65 | NH-piperidin-4-yl-N-benzyl | N(ethyl)-ethyl-N(CH₃)₂ | Piperidin-1-yl | C34 H54 N6 O4 S2 | 675,0 | 675,4 | |
| 66 | NH-ethyl-phenyl-3,4-(OCF₃)₂ | N(ethyl)-ethyl-N(CH₃)₂ | Piperidin-1-yl | C32 H51 N5 O6 S2 | 665,9 | 666,3 | |
| 67 | NH-ethyl-phenyl-4-Cl | N(ethyl)-ethyl-N(CH₃)₂ | Piperidin-1-yl | C30 H46 Cl N5 O4 S2 | 640,3 | 640,3 | |
| 68 | NH-ethyl-phenyl-4-NH₂ | N(ethyl)-ethyl-N(CH₃)₂ | Piperidin-1-yl | C30 H48 N6 O4 S2 | 620,9 | 621,3 | |
| 69 | NH-propyl-phenyl | N(ethyl)-ethyl-N(CH₃)₂ | Piperidin-1-yl | C31 H49 N5 O4 S2 | 619,9 | 620,3 | |
| 70 | NH-butyl-phenyl | N(ethyl)-ethyl-N(CH₃)₂ | Piperidin-1-yl | C32 H51 N5 O4 S2 | 633,9 | 634,3 | |
| 71 | N(CH₃)-ethyl-phenyl | N(ethyl)-ethyl-N(CH₃)₂ | Piperidin-1-yl | C31 H49 N5 O4 S2 | 619,9 | 620,3 | |
| 72 | Piperazin-1-yl-4-ethyl-phenyl | N(ethyl)-ethyl-N(CH₃)₂ | Piperidin-1-yl | C34 H54 N6 O4 S2 | 675,0 | 675,4 | |
| 73 | NH-CH₂-3,5-(CH₃)₂-phenyl | N(ethyl)-ethyl-N(CH₃)₂ | Piperidin-1-yl | C31 H49 N5 O4 S2 | 619,9 | 620,3 | |
| 74 | NH-ethyl-3-CF₃-phenyl | N(ethyl)-ethyl-N(CH₃)₂ | Piperidin-1-yl | C31 H46 F3 N5 O4 S2 | 673,9 | 674,3 | |
| 76 | NH-ethyl-O-phenyl | N(ethyl)-ethyl-N(CH₃)₂ | Piperidin-1-yl | C30 H47 N5 O5 S2 | 621,9 | 622,3 | |
| 77 | NH-ethyl-3,4-Cl₂-phenyl | N(ethyl)-ethyl-N(CH₃)₂ | Piperidin-1-yl | C30 H45 C12 N5 O4 S2 | 674,8 | 674,2 | |
| 78 | Piperazin-1-yl-4-phenyl | NH-ethyl-N-pyrrolidinyl | Piperidin-1-yl | C32 H48 N6 O4 S2 | 644,9 | 645,3 | |
| 79 | Piperidin-1-yl-4-phenyl | NH-ethyl-N-pyrrolidinyl | Piperidin-1-yl | C33 H49 N5 O4 S2 | 643,9 | 644,3 | |
| 80 | Piperidin-1-yl-4-benzyl | NH-ethyl-N-pyrrolidinyl | Piperidin-1-yl | C34 H51 N5 O4 S2 | 657,9 | 658,3 | |
| 81 | NH-ethyl-3,4(OCH₃)₂-phenyl | NH-ethyl-N-pyrrolidinyl | Piperidin-1-yl | C32 H49 N5 O6 S2 | 663,9 | 664,3 | |
| 82 | NH-propyl-phenyl | NH-ethyl-N-pyrrolidinyl | Piperidin-1-yl | C31 H47 N5 O4 S2 | 617,9 | 618,3 | |
| 83 | NH-butyl-phenyl | NH-ethyl-N-pyrrolidinyl | Piperidin-1-yl | C32 H49 N5 O4 S2 | 631,9 | 632,3 | |
| 84 | Piperazin-1-yl-4-ethyl-phenyl | NH-ethyl-N-pyrrolidinyl | Piperidin-1-yl | C34 H52 N6 O4 S2 | 673,0 | 673,4 | |
| 85 | NH-CH₂-(3-CL-phenyl) | NH-ethyl-N-pyrrolidinyl | Piperidin-1-yl | C29 H42 Cl N5 04 S2 | 624,3 | 624,2 | |
| 87 | NH-ethyl-O-phenyl | NH-ethyl-N-pyrrolidinyl | Piperidin-1-yl | C30 H45 N5 O5 S2 | 619,8 | 620,3 | |
| 88 | Piperazin-1-yl-4-CH₃ | NH-ethyl-N(ethyl)₂ | NH-phenyl | C29 H40 N6 O4 S2 | 600,8 | 601,3 | |
| 89 | N(CH₃)-benzyl | NH-ethyl-N(CH₃)₂ | NH-ethyl-N(ethyl)₂ | C30 H53 N7 O4 S2 | 639,9 | 640,4 | |
| 90 | N(CH₃)-benzyl | Piperazin-1-yl-4-CH₃ | NH-ethyl-N(ethyl)₂ | C31 H53 N7 O4 S2 | 651,9 | 652,4 | |
| 91 | Piperazin-1-yl-4-phenyl | NH-ethyl-N(CH₃)₂ | Piperidin-1-yl | C30 H46 N6 O4 S2 | 618,9 | 619,3 | |
| 92 | Piperidin-1-yl-4-phenyl | NH-ethyl-N(CH₃)₂ | Piperidin-1-yl | C31 H47 N5 O4 S2 | 617,9 | 618,3 | |
| 93 | Piperidin-1-yl-4-benzyl | NH-ethyl-pyrrolidinyl | NH-(CH₂)₃-O-CH₃ | C32 H51 N5 O6 S2 | 665,9 | 666,3 | |
| 94 | NH-ethyl-3,4-(OCH₃)₂-phenyl | N(ethyl)-ethyl-N(CH₃)₂ | NH-ethyl-thien-2-yl | C34 H47 N5 O6 S4 | 750,0 | 750,2 | |
| 95 | Piperidin-1-yl-4-phenyl | N(ethyl)-ethyl-N(CH₃)₂ | NH-ethyl-thien-2-yl | C35 H47 N5 O4 S4 | 730,1 | 730,3 | |
| 96 | Piperazin-1-yl-4-phenyl | N(ethyl)-ethyl-N(CH₃)₂ | NH-ethyl-thien-2-yl | C34 H46 N6 O4 S4 | 731,0 | 731,3 | |
| 97 | NH-CH₂-naphth-1-yl | NH-ethyl-N-pyrrolidinyl | NH-ethyl-thien-2-yl | C35 H41 N5 O4 S4 | 724,0 | 724,2 | |
| 98 | Piperazin-1-yl-4-phenyl | N(ethyl)-ethyl-N(CH₃)₂ | NH-propyl-O-CH₃ | C30 H50 N6 O6 S2 | 654,9 | 655,3 | |
| 99 | NH-(4-t-butyl)-benzyl | NH-ethyl-N-pyrrolidinyl | NH-ethyl-thien-2-yl | C35 H47 N5 O4 S4 | 730,1 | 730,3 | |
| 100 | NH-(3,4-Cl₂)-benzyl | NH-ethyl-N(CH₃)₂ | Piperidin-1-yl | C27 H39 Cl2 N5 O4 S2 | 632,7 | 632,2 | |
| 101 | NH-(3-Cl)-benzyl | NH-ethyl-N(CH₃)₂ | Piperidin-1-yl | C27 H40 Cl N5 O4 S2 | 598,2 | 598,2 | |
| 102 | NH-(4-t-butyl)-benzyl | NH-ethyl-N(CH₃)₂ | Piperidin-1-yl | C31 H49 N5 O4 S2 | 619,9 | 620,3 | |
| 103 | NH-ethyl-O-phenyl | NH-ethyl-N(CH₃)₂ | Piperidin-1-yl | C28 N43 N5 O5 S2 | 593,8 | 594,3 | |
| 104 | Piperazin-1-yl-4-phenyl | NH-ethyl-N-pyrrolidinyl | NH-(CH₂)₃-O-CH₃ | C30 H48 N6 O6 S2 | 652,9 | 653,3 | |
| 105 | NH-(4-t-butyl)-benzyl | NH-ethyl-N-pyrrolidinyl | NH-(CH₂)₃-O-CH₃ | C31 H51 N5 O6 S2 | 653,9 | 654,3 | |
| 106 | Piperazin-1-yl-4-phenyl | NH-ethyl-N(CH₃)₂ | NH-(CH₂)₃-O-CH₃ | C28 H46 N6 O6 S2 | 626,8 | 627,3 | |
| 107 | NH-(4-t-butyl)-benzyl | NH-ethyl-N(CH₃)₂ | NH-(CH₂)₃-O-CH₃ | C29 H49 N5 O6 S2 | 627,9 | 628,3 | |
| 108 | Piperazin-1-yl-4-phenyl | 4-N(CH₃)-piperazin-1-yl | NH-(CH₂)₃-O-CH₃ | C29 H46 N6 O6 S2 | 638,9 | 639,3 | |
| 109 | NH-(4-t-butyl)-benzyl | 4-N(CH₃)-piperazin-1-yl | NH-(CH₂)₃-O-CH₃ | C30 H49 N5 O6 S2 | 639,9 | 640,3 | |
| 110 | 1,2,5,6-tetrahydropyridin-1-yl-(4-phenyl) | 4-N(CH₃)-piperazin-1-yl | N(CH₃)-phenyl | C36 H41 N5 O4 S2 | 671,9 | 672,3 | |

Die Verbindungen der Formel I zeichnen sich durch günstige Wirkungen auf den Fettstoffwechsel aus, insbesondere sind sie als Hypolipidämika geeignet. Die Verbindungen können allein oder in Kombination mit weiteren Lipidsenkern eingesetzt werden. Solche weiteren Lipidsenker werden z.B. in der Roten Liste, Kapitel 58 genannt. Die Verbindungen eignen sich zur Prophylaxe sowie insbesondere zur Behandlung von Hyperlipidämie.

Arteriosklerose ist eine komplexe Erkrankung des Stoffwechsel- und Kreislaufsystems. Erhöhtes Plasma-LDL-Cholesterin ist einer der Hauptrisikoparameter dieser Erkankung. Beim Menschen wird LDL-Cholesterin zum überwiegenden Teil über den LDL-Rezeptor in der Leber aus dem Blutkreislauf entfernt. Eine Senkung des LDL-Plasmacholesterins senkt das Arterioskleroserisiko und damit auch die Gesamtmortalität. Die erfindungsgemäßen Verbindungen eignen sich somit auch zur Prophylaxe und zur Behandlung arteriosklerotischer Erkrankungen.

Die Wirksamkeit der Verbindungen wurde wie folgt getestet:

### 1) In-vitro-Bestimmung des LDL-Rezeptorinduktion mittels des Luziferase-Assays

Die LDL-Rezeptorinduktion wird mittels des Luziferase Tests folgendermaßen bestimmt: Dazu wird ein regulatorisches DNA Fragment (4kb) des humanen LDL-Rezeptors Gens , daß die komplette Promotorregion enthält, an das Luziferase-Reporter-Gen aus der Feuerfliege gekoppelt und stabil in eine Hep-G2 Zelllinie transfiziert. Zellen aus dieser Linie wurden auf Kollagen beschichteten 96 Well Platten in MEM (Minimum Essential Medium) ausgesäht. Nach 24 Stunden in Kultur wurden die Testsubstanzen, gelöst in DMSO, in Endkonzentrationen von 10 nM bis 10 µM zugegeben( DMSO Endkonzentration = 2%). Die Substanzen wurden über Nacht für 12-18 Stunden inkubiert (jeweils 4 Wells/Konz.), danach wurde D-Luziferin als Substrat für die Luziferase zugegeben und die Lumineszens gemessen. Die gemessene Luminiszens in prozentualer Relation zu Kontrolle gesetzt (Kontrolle = 100%), die nur mit DMSO inkubiert wurde, ergibt das Maß für die relative LDL-Rezeptorinduktion (Tabelle 2).

Weitere Details der Methode sind beschrieben in: Current Protocols in Molecular Biology, F.M. Ausubel, R. Brent, R.E.Kingston, D.D. Moore, J.G.Seidman, J.A.Smith and Kevin Struhl editors, J.Wiley and Sons Inc., U.S.A.

**Tabelle 2: LDL-Rezeptor Induktion ausgewählter Beispiele in % gegenüber der Kontrolle**

| Beispiel | LDL-Rezeptor Induktion (% gegen Kontrolle) |
|---|---|
| 1 | 195 % (1,5 µM) |
| 2 | 227 % (4 µM); 155 % (1,5 µM) |
| 3 | 414 % (1,5 µM), 184 % (0,15 µM) |
| 5 | 240 % (4 µM) |
| 6 | 299 % (4 µM) |
| 8 | 190% (4 µM) |
| 9 | 402 % (1,5 µM), 244% (0,15 µM) |
| 10 | 270 % (4 µM), 144 % (0,15 µM) |
| 12 | 244 % (1,5 µM) |
| 15 | 243 % (1,5 µM), 137 % (0,15 µM) |
| 16 | 214 % (1,5 µM) |
| 25 | 189 % (1,5 µM), 168 % (0,15 %) |
| 26 | 206 % (1,5 µM), 146 % (0,15 µM) |
| 46 | 223 % (1,5 µM), 152 % (0,15 µM) |
| 58 | 219 % (4 µM); 196 % (1,5 µM) |
| 61 | 190 % (4 µM) |
| 65 | 314 % (4 µM), 292 % (1,5 µM), 177 % (0,15 µM) |
| 70 | 238 % (4 µM), 194 % (0,15 µM) |
| 84 | 223 % (4 µM), 199 % (1,5 µM) |
| 92 | 233 % (4 µM), 213 % (1,5 µM) |
| 93 | 337 % (4 µM), 293 % (1,5 µM), 213 % (0,15 µM) |
| 94 | 321 % (4 µM), 194 % (1,5 µM) |
| 95 | 301 % (4 µM), 267 % (1,5 µM), 198 % (0,15 µM) |
| 96 | 326 % (4 µM), 278 % (1,5 µM), 138 % (0,15 µM) |
| 98 | 285 % (4µM), 249 (1,5 µM) |
| 99 | 322 % (4 µM), 247 % (1,5 µM) |
| 102 | 298 % (4 µM), 239 % (1,5 µM) |
| 108 | 249 % (4 µM), 191 % (1,5 µM) |

### 2) In vivo-Bestimmung der LDL-Cholesterinsenkung am Hamster; Cholesterinsenkenden Wirkung von LDL-Rezeptorinduktoren bei hyperlipämischen Hamstern

In diesem Tierversuch wurde die Wirkung der LDL-Rezeptorinduktoren nach Bolusapplikation am fettgefütterten Hamster untersucht.

Als Versuchstiere wurden männliche syrische Hamster (Charles River) mit einem durchschnittlichen Körpergewicht von 100 - 120 g zu Adaptionsbeginn verwendet. Die Tiere wurden anhand des Körpergewichtes in Gruppen ( n = 6 ) eingeteilt. Durch Fütterung einer mit 15 % Butter und 3 % Cholesterin angereicherten Diät wurde eine starke Hyperlipidämie induziert. Nach 2 wöchiger Vorfütterung begann die Behandlung. Die Testsubstanzen wurden oral mit einer Schlund-Magensonde über einen Zeitraum von 10 Tagen 1 mal täglich appliziert. Nach 10 Tagen wurde der Plasma Lipidspiegel analysiert.

Tabelle 3 zeigt die relativen Veränderungen des Lipidspiegels im Vergleich zu Plazebo behandelten Kontrolltieren in %.

**Tabelle 3**

| Relative Veränderung des Plasma-Lipidspiegels bei hyperlipidämischen Hamstern nach zehntägiger oraler Behandlung [%] | | | | | | |
|---|---|---|---|---|---|---|
| Gruppe | Behandlung; | | Total-Cholesterin | LDL-Cholesterin | | Triglyceride |
| | (Bsp.-Nr./Dosis) | | | | | |
| 1 | Kontrolle I | | - | - | | - |
| 2 | 95 | | - 45 | - 44 | | - 61 |
| | 20 mg/kg p.o. | | | | | |
| 3 | 95 | | - 50 | - 49 | | - 73 |
| | 40 mg/kg p.o. | | | | | |
| 4 | 98 | | -23 | - 26 | | - 27 |
| | 20 mg/kg p.o. | | | | | |
| 5 | 98 | | -46 | - 44 | | - 68 |
| | 40 mg/kg p.o. | | | | | |
| | | | | | | |

Aus der deutlichen Senkung von Total-Cholesterin, LDL-Cholesterin und Triglyceriden ist die gute lipidsenkende Wirkung der erfindungsgemäßen Verbindungen abzulesen.

Zur näheren Erläuterung der Herstellung ist nachfolgend ein Beispiel (Nr. 64) genau beschrieben.

### Ausführungsbeispiel:

### N-Ethyl-N',N'-dimethyl-N-[5-(4-phenyl-piperazin-1-yl)-2,4-bis-(piperidin-1-sulfonyl)-phenyl]-ethan-1,2-diamin (Tabelle 1, Beispiel 64)

2,63 g (5,1 mmol) N-[5-Chloro-2,4-bis-(piperidin-1-sulfonyl)-phenyl]-N-ethyl-N',N'-dimethyl-ethan-1,2-diamin, hergestellt wie u.a. beschrieben, werden in 12 ml 4-Phenylpiperazin gelöst und das Reaktionsgemisch 9 Stunden bei 90 °C gerührt. Zur Aufarbeitung wird mittels Ethylacetat/Wasser extrahiert, die vereinigten organischen Phasen über Natriumsulfat getrocknet und das Extraktionsmittel unter reduziertem Druck am Rotationsverdampfer entfernt. Anschließend wird durch Chromatographie an Kieselgel (40 - 63 µ, Fa. Merck Darmstadt; mobile Phase Dichlormethan/ Methanol = 20 / 1) gereinigt.

Das Reaktionsprodukt, Ausbeute 1,43 g ( 44 %), kristallisiert aus einer Diisopropylether/n-Pentan-Lösungsmittelmischung in Form hellgelber Kristalle vom Schmelzpunkt 116 bis 118 °C .
C32 H50 N6 04 S2 (646,9); Massenspektrum 647,3 (M+H⁺)

### Synthese von N-[5-Chloro-2,4-bis-(piperidin-1-sulfonyl)-phenyl]-N-ethyl-N',N'-dimethyl-ethan-1,2-diamin

11,2 g (25,3 mmol) 4,6-Dichloro-benzen-1,3-disulfonsäure-bis-piperidylamid, (hergestellt wie u.a. beschrieben), werden in 100 ml Ethanol gelöst und mit 4,26 g N,N-Dimethyl-N'-ethylendiamin versetzt. Das Reaktionsgemisch wird 12 Stunden unter Rückfluß des Lösungsmittels erhitzt. Anschließend wird auf 500 ml Eiswasser gegossen und dreimal mit jeweils 100 ml Ethylacetat extrahiert. Nach Vereinigung der organischen Phasen, Trocknung dieser mittels Natriumsulfat und Entfernen des Extraktionsmittels unter reduziertem Druck am Rotationsverdampfer wird mittels Chromatographie an Kieselgel (40 - 63 µ, Fa. Merck Darmstadt; mobile Phase Dichlor-methan / Ethylaceat = 20 : 1) gereinigt.
Man erhält nach Entfernen des Lösungsmittels 6,8 g N-[5-Chloro-2,4-bis-(piperidin-1-sulfonyl)-phenyl]-N-ethyl-N',N'-dimethyl-ethan-1,2-diamin,
Ausbeute 52 % der Theorie, farbloses Öl.
C₂₂ H ₃₇ Cl N₄ O₄ S₂ (521,1), Massenspektrum 521,2 (M+H⁺)

### Synthese von 4,6-Dichloro-benzen-1,3-disulfonsäure-bis-piperidylamid

20 g (58 mmol) 4,6-Dichloro-benzen-1,3-disulfonyl dichlorid (hergestellt wie u.a. beschrieben) werden in 175 ml absolutem Tetrahydrofuran gelöst und unter Eiskühlung bei 0°C tropfenweise mit einem Gemisch aus 12,65 ml Piperidin, 16,1 ml Triethylamin und 10 ml Tetrahydrofuran versetzt. Die Reaktionstemperatur darf dabei maximal auf Zimmertemperatur ansteigen. Anschließend wird eine Stunde bei Zimmertemperatur nachgerührt und vom gebildeten Niederschlag abfiltriert. Nach Trocknung mittels Natriumsulfat und Einengen der Mutterlauge unter reduziertem Druck wird mittels Chromatographie an Kieselgel (40 - 63 µ, Fa. Merck Darmstadt) mit n-Heptan: Ethylacetat = 1:2 als mobiler Phase gereinigt.
Man erhält 18,2 g (71,1 % d. Th.) 4,6-Dichloro-benzen-1,3-disuffonsäure-bis-piperidylamid vom Schmelzpunkt 170 °C.

### Synthese von 4,6-Dichloro-benzen-1,3-disulfonyl dichlorid

80 g (0,54 mol) 1,3-Dichlorbenzol werden in 645 g Chlorsulfonsäure gelöst und 5 Stunden bei 125 °C gerührt. Anschließend wird 8 Stunden bei 23°C gerührt. Unter Kühlung werden anschließend 145,6 ml Thionylchlorid zugegeben und dann 2 Stunden bei 80 °C gerührt. Zur Hydrolyse wird die Reaktionmischung dann vorsichtig und unter starker Kühlung tropfenweise mit 42 ml Wasser versetzt. Anschließend wird die Reaktionslösung bei 0°C auf ca. 3,5 l Eis/Wasser-Gemisch getropft. Das Reaktionsprodukt fällt dabei als ein farbloser Feststoff aus, welcher anschließend abfiltriert wird.

Man erhält nach Trocknung 179,2 g 4,6-Dichloro-benzen-1,3-disulfonyl dichlorid (95 % d. Th.) vom Schmelzpunkt 104 °C; das Produkt schmilzt unter Zersetzung.

## Patentansprüche

1. Verwendung der Verbindungen der Formel I, 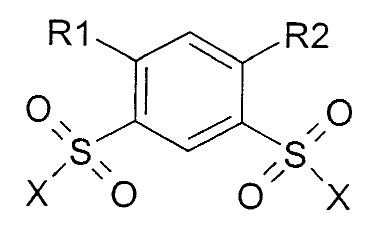 worin bedeuten
X, R1, R2 unabhängig voneinander NR6R7, Pyrrolidin, Piperidin, Piperazin, Morpholin, Tetrahydropyridin, wobei die Ringe jeweils substituiert sein können mit Phenyl, (C₁-C₆)-Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl, wobei der Phenyl-Substituent unsubstituiert oder bis zu zweifach substituiert ist mit F, Cl, Br, OH, CF₃, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, CONH(C₃-C₆)Cycloalkyl, NH₂, NH-CO-(C₁-C₆)-Alkyl, NH-CO-Phenyl;
R6 und R7 unabhängig voneinander H, (C₁-C₆)-Alkyl, (C₁-C₆)-Alkyl-O-(C₁-C₆)-Alkyl,O-(C₁-C₆)-Alkyl ,(C₃-C₆)-Cycloalkyl, CO-(C₁-C₆)-Alkyl, (C₁-C₆)-Alkyl-NH-C(O)-(C₁-C₆)-Alkyl, (C₁-C₆)-Alkyl-NH-(C₁-C₆)-Alkyl, (C₁-C₆)-Alkyl-N-[(C₁-C₆)-Alkyl]₂, (C₁-C₆)-Alkyl-O-Phenyl, CHO, CO-Phenyl, (CH₂)ₙ-Ar, wobei n = 0 - 6 sein kann und Ar gleich Phenyl, Biphenylyl, 1- oder 2-Naphthyl, 1- oder 2-Tetrahydrofuranyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3-, 4- oder 5-Isoxazolyl, (C₃-C₆)-Cycloalkyl, Piperidinyl, Pyrrolidinyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2-, 3- oder 4-Morpholinyl, 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und Ar zu zweifach mit F, Cl, Br, OH, CF₃, NO₂, CN, OCF₃, O-CH₂-O, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, CONH(C₃-C₆)Cycloalkyl, NH₂, NH-CO-(C₁-C₆)-Alkyl, NH-CO-Phenyl, Pyrrolidin-1-yl, Morpholin-1-yl, Piperidin-1-yl, Piperazin-1-yl, 4-Methyl-piperazin-1-yl, (CH₂)ₙ-Phenyl, O-(CH₂)ₙ-Phenyl, S-(CH₂)ₙ-Phenyl, SO₂-(CH₂)ₙ-Phenyl, wobei n = 0-3, substituiert sein kann;
sowie derer physiologisch verträglichen Salze und physiologisch funktionellen Derivate zur Herstellung eines Medikamente zur Prävention und Behandlung von Hyperlipidämie.

2. Verwendung der Verbindungen der Formel I, gemäß Anspruch 1, **dadurch gekennzeichnet, daß** darin bedeuten
R1 NR6R7, Pyrrolidin, Piperidin, Piperazin, Tetrahydropyridin, wobei die Ringe jeweils substituiert sein können mit Phenyl, (C₁-C₆)-Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl, wobei der Phenyl-Substituent unsubstituiert oder bis zu zweifach substituiert ist mit F, Cl, Br, CF₃, CN, OCF₃, O-(C₁-C₆)-Alkyl, S-(C₁-C₆)-Alkyl, (C₁-C₆)-Alkyl, (C₃-C₆)-Cycloalkyl, COOH, COO(C₁-C₆)-Alkyl, COO(C₃-C₆)Cycloalkyl, CONH₂, CONH(C₁-C₆)Alkyl, CON[(C₁-C₆)Alkyl]₂, NH₂, NH-CO-(C₁-C₆)-Alkyl, NH-CO-Phenyl;
R6, R7 unabhängig voneinander H, (C₁-C₆)-Alkyl, (C₁-C₆)-Alkyl-O-(C₁-C₆)-Alkyl, (C₃-C₆)-Cycloalkyl, CO-(C₁-C₆)-Alkyl, (C₁-C₆)-Alkyl-NH-C(O)-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, Biphenylyl, 1- oder 2-Naphthyl, 2-, 3- oder 4-Pyridyl, 2- oder 3-Thienyl, 2-, 4- oder 5-Thiazolyl, 2-, 4- oder 5-Oxazolyl, 3- oder 5-Isoxazolyl, (C₃-C₆)-Cycloalkyl, Piperidinyl, Pyrrolidinyl, 2-, 4- oder 5-Pyrimidinyl, 2-, 3- oder 4-Morpholinyl, 2- oder 5-Benzimidazolyl, 2-Benzothiazolyl oder Indol-3-yl, Indol-5-yl sein kann und Ar bis zu zweifach substituiert sein kann mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, NH₂, NH-CO-Phenyl, (CH₂)ₙ-Phenyl, O-(CH₂)ₙ-Phenyl, S-(CH₂)ₙ-Phenyl, wobei n = 0-3, substituiert sein kann;
R2 NR8R9, Piperazin, wobei Piperazin substituiert sein kann mit (C₁-C₆)-Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl;
R8, R9 unabhängig voneinander H, (C₁-C₆)-Alkyl, (C₃-C₆)-Cycloalkyl, CO-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 2-, 3- oder 4-Pyridyl, Piperidinyl, Pyrrolidinyl, Morpholinyl sein kann;
X NR10R11, Pyrrolidin, Piperidin, Piperazin, Morpholin, wobei die Ringe jeweils substituiert sein können mit Phenyl, (C₁-C₆)Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl, wobei der Phenyl-Substituent unsubstituiert oder bis zu zweifach substituiert ist mit F, Cl, Br, CF₃, CN, OCF₃, O-(C₁-C₆)-Alkyl, (C₁-C₆)-Alkyl, (C₃-C₆)-Cycloalkyl, COOH, COO(C₁-C₆)-Alkyl, COO(C₃-C₆)Cycloalkyl, CONH₂, CONH(C₁-C₆)Alkyl, CON[(C₁-C₆)Alkyl]₂, NH₂, NH-CO-(C₁-C₆)-Alkyl, NH-CO-Phenyl;
R10, R11 unabhängig voneinander H, (C₁-C₆)-Alkyl, , (C₁-C₆)-Alkyl-O-(C₁-C₆)-Alkyl, (C₃-C₆)-Cycloalkyl, CO-(C₁-C₆)-Alkyl, (C₁-C₆)-Alkyl-NH-(C₁-C₆)-Alkyl, (C₁-C₆)-Alkyl-N-[(C₁-C₆)-Alkyl]₂, CO-Phenyl, (CH₂)ₙ-Ar, wobei n = 0 - 6 sein kann und Ar gleich Phenyl, Biphenyl, 1- oder 2-Naphthyl, 1- oder 2-Tetrahydrofuranyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 3- oder 5-Isoxazolyl, Piperidinyl, Pyrrolidinyl, 2-, 4- oder 5-Pyrimidinyl, 2-, 3- oder 4-Morpholinyl, 2-Benzothiazolyl sein kann und Ar zweifach substituiert sein kann mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, CONH₂, CONH(C₁-C₆)Alkyl, CON[(C₁-C₆)Alkyl]₂, NH-CO-(C₁-C₆)-Alkyl, NH-CO-Phenyl, (CH₂)ₙ-Phenyl, wobei n = 0-3, substituiert sein kann;
sowie derer physiologisch verträglichen Salze und physiologisch funktionellen Derivate zur Herstellung eines Medikamente zur Prävention und Behandlung von Hyperlipidämie.

3. Verwendung der Verbindungen der Formel I, gemäß Anspruch 1 oder 2, **dadurch gekennzeichnet, daß** darin bedeuten
R1 NR6R7, Piperidin, Piperazin, Tetrahydropyridin, wobei die Ringe jeweils substituiert sein können mit Phenyl, (C₁-C₆)-Alkyl-Phenyl;
R6, R7 unabhängig voneinander H, (C₁-C₆)-Alkyl, (C₁-C₆)-Alkyl-O-(C₁-C₆)-Alkyl, (C₃-C₆)-Cycloalkyl, (C₁-C₆)-Alkyl-NH-C(O)-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 1- oder 2-Naphthyl, 2-, 3- oder 4-Pyridyl, (C₃-C₆)-Cycloalkyl, Piperidinyl, Pyrrolidinyl, 2-, 4- oder 5-Pyrimidinyl, 2-, 3- oder 4-Morpholinyl sein kann und Ar bis zu zweifach substituiert sein kann mit F, Cl, Br, OH, CF₃, NO₂, CN, OCF₃, O-(C₁-C₆)-Alkyl, (C₁-C₆)-Alkyl, NH₂;
R2 NR8R9, Piperazin, wobei Piperazin substituiert sein kann mit (C₁-C₆)-Alkyl;
R8, R9 unabhängig voneinander H, (C₁-C₆)-Alkyl, (C₃-C₆)-Cycloalkyl, CO-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 2-, 3- oder 4-Pyridyl, Piperidinyl, Pyrrolidinyl, Morpholinyl sein kann;
X NR1 OR11, Pyrrolidin, Piperidin, Morpholin, wobei die Ringe jeweils substituiert sein können mit Phenyl, (C₁-C₆)-Alkyl-Phenyl;
R10, R11 unabhängig voneinander H, (C₁-C₆)-Alkyl, (C₁-C₆)-Alkyl-O-(C₁-C₆)-Alkyl, (C₃-C₆)-Cycloalkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 2- oder 3-Thienyl;
sowie derer physiologisch verträglichen Salze zur Herstellung eines Medikaments zur Prävention und Behandlung von Hyperlipidämie.

4. Verwendung der Verbindungen gemäß einem oder mehreren der Ansprüche 1 bis 3 in Kombination mit einem oder mehreren Lipidsenkern zur Herstellung eines Medikaments zur Prophylaxe oder Behandlung von Hyperlipidämie.

5. Verwendung der Verbindungen der Formel I, 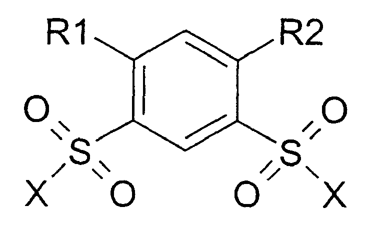 worin bedeuten
X, R1, R2 unabhängig voneinander NR6R7, Pyrrolidin, Piperidin, Piperazin, Morpholin, Tetrahydropyridin, wobei die Ringe jeweils substituiert sein können mit Phenyl, (C₁-C₆)-Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl, wobei der Phenyl-Substituent unsubstituiert oder bis zu zweifach substituiert ist mit F, Cl, Br, OH, CF₃, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, CONH(C₃-C₆)Cycloalkyl, NH₂, NH-CO-(C₁-C₆)-Alkyl, NH-CO-Phenyl;
R6 und R7 unabhängig voneinander H, (C₁-C₆)-Alkyl, (C₁-C₆)-Alkyl-O-(C₁-C₆)-Alkyl,O-(C₁-C₆)-Alkyl ,(C₃-C₆)-Cycloalkyl, CO-(C₁-C₆)-Alkyl, (C₁-C₆)-Alkyl-NH-C(O)-(C₁-C₆)-Alkyl, (C₁-C₆)-Alkyl-NH-(C₁-C₆)-Alkyl, (C₁-C₆)-Alkyl-N-[(C₁-C₆)-Alkyl]₂, (C₁-C₆)-Alkyl-O-Phenyl, CHO, CO-Phenyl,
(CH₂)ₙ-Ar, wobei n = 0 - 6 sein kann und Ar gleich Phenyl, Biphenylyl, 1- oder 2-Naphthyl, 1- oder 2-Tetrahydrofuranyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3-, 4- oder 5-Isoxazolyl, (C₃-C₆)-Cycloalkyl, Piperidinyl, Pyrrolidinyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2-, 3- oder 4-Morpholinyl, 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl sein kann und Ar zu zweifach mit F, Cl, Br, OH, CF₃, NO₂, CN, OCF₃, O-CH₂-O, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, CONH(C₃-C₆)Cycloalkyl, NH₂, NH-CO-(C₁-C₆)-Alkyl, NH-CO-Phenyl, Pyrrolidin-1-yl, Morpholin-1-yl, Piperidin-1-yl, Piperazin-1-yl, 4-Methyl-piperazin-1-yl, (CH₂)ₙ-Phenyl, O-(CH₂)ₙ-Phenyl, S-(CH₂)ₙ-Phenyl, SO₂-(CH₂)ₙ-Phenyl, wobei n = 0-3, substituiert sein kann;
sowie derer physiologisch verträglichen Salze und physiologisch funktionellen Derivate zur Herstellung eines Medikamente zur Prävention und Behandlung von Arteriosklerose.

6. Verwendung der Verbindungen der Formel I, gemäß Anspruch 5, **dadurch gekennzeichnet, daß** darin bedeuten
R1 NR6R7, Pyrrolidin, Piperidin, Piperazin, Tetrahydropyridin, wobei die Ringe jeweils substituiert sein können mit Phenyl, (C₁-C₆)-Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl, wobei der Phenyl-Substituent unsubstituiert oder bis zu zweifach substituiert ist mit F, Cl, Br, CF₃, CN, OCF₃, O-(C₁-C₆)-Alkyl, S-(C₁-C₆)-Alkyl, (C₁-C₆)-Alkyl, (C₃-C₆)-Cycloalkyl, COOH, COO(C₁-C₆)-Alkyl, COO(C₃-C₆)Cycloalkyl, CONH₂, CONH(C₁-C₆)Alkyl, CON[(C₁-C₆)Alkyl]₂, NH₂, NH-CO-(C₁-C₆)-Alkyl, NH-CO-Phenyl;
R6, R7 unabhängig voneinander H, (C₁-C₆)-Alkyl, (C₁-C₆)-Alkyl-O-(C₁-C₆)-Alkyl, (C₃-C₆)-Cycloalkyl, CO-(C₁-C₆)-Alkyl, (C₁-C₆)-Alkyl-NH-C(O)-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, Biphenylyl, 1- oder 2-Naphthyl, 2-, 3- oder 4-Pyridyl, 2- oder 3-Thienyl, 2-, 4- oder 5-Thiazolyl, 2-, 4- oder 5-Oxazolyl, 3- oder 5-Isoxazolyl, (C₃-C₆)-Cycloalkyl, Piperidinyl, Pyrrolidinyl, 2-, 4- oder 5-Pyrimidinyl, 2-, 3- oder 4-Morpholinyl, 2- oder 5-Benzimidazolyl, 2-Benzothiazolyl oder Indol-3-yl, Indol-5-yl sein kann und Ar bis zu zweifach substituiert sein kann mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, NH₂, NH-CO-Phenyl, (CH₂)ₙ-Phenyl, O-(CH₂)ₙ-Phenyl, S-(CH₂)ₙ-Phenyl, wobei n = 0-3, substituiert sein kann;
R2 NR8R9, Piperazin, wobei Piperazin substituiert sein kann mit (C₁-C₆)-Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl;
R8, R9 unabhängig voneinander H, (C₁-C₆)-Alkyl, (C₃-C₆)-Cycloalkyl, CO-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 2-, 3- oder 4-Pyridyl, Piperidinyl, Pyrrolidinyl, Morpholinyl sein kann;
X NR10R11, Pyrrolidin, Piperidin, Piperazin, Morpholin, wobei die Ringe jeweils substituiert sein können mit Phenyl, (C₁-C₆)Alkyl-Phenyl, (C₁-C₆)-Alkyl, (C₁-C₆)-Alkyl-OH, O-Phenyl, S-Phenyl,(CO)-(C₁-C₆)-Alkyl, (CO)-Phenyl, wobei der Phenyl-Substituent unsubstituiert oder bis zu zweifach substituiert ist mit F, Cl, Br, CF₃, CN, OCF₃, O-(C₁-C₆)-Alkyl, (C₁-C₆)-Alkyl, (C₃-C₆)-Cycloalkyl, COOH, COO(C₁-C₆)-Alkyl, COO(C₃-C₆)Cycloalkyl, CONH₂, CONH(C₁-C₆)Alkyl, CON[(C₁-C₆)Alkyl]₂, NH₂, NH-CO-(C₁-C₆)-Alkyl, NH-CO-Phenyl;
R10, R11 unabhängig voneinander H, (C₁-C₆)-Alkyl, , (C₁-C₆)-Alkyl-O-(C₁-C₆)-Alkyl, (C₃-C₆)-Cycloalkyl, CO-(C₁-C₆)-Alkyl, (C₁-C₆)-Alkyl-NH-(C₁-C₆)-Alkyl, (C₁-C₆)-Alkyl-N-[(C₁-C₆)-Alkyl]₂, CO-Phenyl, (CH₂)ₙ-Ar, wobei n = 0 - 6 sein kann und Ar gleich Phenyl, Biphenylyl, 1- oder 2-Naphthyl, 1- oder 2-Tetrahydrofuranyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 3- oder 5-Isoxazolyl, Piperidinyl, Pyrrolidinyl, 2-, 4- oder 5-Pyrimidinyl, 2-, 3- oder 4-Morpholinyl, 2-Benzothiazolyl sein kann und Ar zweifach substituiert sein kann mit F, Cl, Br, OH, CF₃, NO₂, CN, OCF₃, O-(C₁-C₆)-Alkyl, S-(C₁-C₆)-Alkyl, SO-(C₁-C₆)-Alkyl, SO₂-(C₁-C₆)-Alkyl, (C₁-C₆)-Alkyl, (C₃-C₆)-Cycloalkyl, CONH₂, CONH(C₁-C₆)Alkyl, CON[(C₁-C₆)Alkyl]₂, NH-CO-(C₁-C₆)-Alkyl, NH-CO-Phenyl, (CH₂)ₙ-Phenyl, wobei n = 0-3, substituiert sein kann;
sowie derer physiologisch verträglichen Salze und physiologisch funktionellen Derivate zur Herstellung eines Medikamente zur Prävention und Behandlung von Arteriosklerose.

7. Verwendung der Verbindungen der Formel I, gemäß Anspruch 5 oder 6, **dadurch gekennzeichnet, daß** darin bedeuten
R1 NR6R7, Piperidin, Piperazin, Tetrahydropyridin, wobei die Ringe jeweils substituiert sein können mit Phenyl, (C₁-C₆)-Alkyl-Phenyl;
R6, R7 unabhängig voneinander H, (C₁-C₆)-Alkyl, (C₁-C₆)-Alkyl-O-(C₁-C₆)-Alkyl, (C₃-C₆)-Cycloalkyl, (C₁-C₆)-Alkyl-NH-C(O)-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 1- oder 2-Naphthyl, 2-, 3- oder 4-Pyridyl, (C₃-C₆)-Cycloalkyl, Piperidinyl, Pyrrolidinyl, 2-, 4- oder 5-Pyrimidinyl, 2-, 3- oder 4-Morpholinyl sein kann und Ar bis zu zweifach substituiert sein kann mit F, Cl, Br, OH, CF₃, NO₂, CN, OCF₃, O-(C₁-C₆)-Alkyl, (C₁-C₆)-Alkyl, NH₂;
R2 NR8R9, Piperazin, wobei Piperazin substituiert sein kann mit (C₁-C₆)-Alkyl;
R8, R9 unabhängig voneinander H, (C₁-C₆)-Alkyl, (C₃-C₆)-Cycloalkyl, CO-(C₁-C₆)-Alkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 2-, 3- oder 4-Pyridyl, Piperidinyl, Pyrrolidinyl, Morpholinyl sein kann;
X NR10R11, Pyrrolidin, Piperidin, Morpholin, wobei die Ringe jeweils substituiert sein können mit Phenyl, (C₁-C₆)-Alkyl-Phenyl;
R10, R11 unabhängig voneinander H, (C₁-C₆)-Alkyl, (C₁-C₆)-Alkyl-O-(C₁-C₆)-Alkyl, (C₃-C₆)-Cycloalkyl, (C₁-C₆)-Alkyl-NH-(C₁-C₆)-Alkyl, (C₁-C₆)-Alkyl-N-[(C₁-C₆)-Alkyl]₂,
(CH₂)ₙ-Ar, wobei n = 0 - 6 sein kann und Ar gleich Phenyl, 2- oder 3-Thienyl;
sowie derer physiologisch verträglichen Salze zur Herstellung eines Medikaments zur Prävention und Behandlung von Arteriosklerose.

8. Verwendung der Verbindungen gemäß einem oder mehreren der Ansprüche 5 bis 7 in Kombination mit einem oder mehreren Lipidsenkern zur Herstellung eines Medikaments zur Prophylaxe oder Behandlung von Arteriosklerose.

9. Verwendung der Verbindungen gemäß einem oder mehreren der Ansprüche 1 bis 3 in Kombination mit einem oder mehreren Lipidsenkern zur Herstellung eines Medikaments zur Prophylaxe oder Behandlung von Hyperlipidämie.

## Claims

1. The use of compounds of formula I, 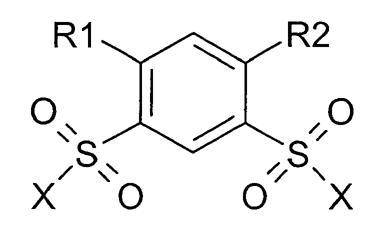 in which
X, R1, R2 are, independently of one another, NR6R7, pyrrolidine, piperidine, piperazine, morpholine, tetrahydropyridine, it being possible for each of the rings to be substituted by phenyl, (C₁-C₆)-alkyl-phenyl, (C₁-C₆)-alkyl, (C₁-C₆)-alkyl-OH, O-phenyl, S-phenyl, (CO)-(C₁-C₆)-alkyl, (CO)-phenyl, where the phenyl substituent is unsubstituted or up to disubstituted by F, Cl, Br, OH, CF₃, CN, OCF₃, O-(C₁-C₆)-alkyl, S-(C₁-C₆)-alkyl, SO-(C₁-C₆)-alkyl, SO₂-(C₁-C₆)-alkyl, (C₁-C₆)-alkyl, (C₃-C₆)-cycloalkyl, COOH, COO(C₁-C₆)alkyl, COO(C₃-C₆)cycloalkyl, CONH₂, CONH(C₁-C₆)alkyl, CON[(C₁-C₆)alkyl]₂, CONH(C₃-C₆)cycloalkyl, NH₂, NH-CO-(C₁-C₆)-alkyl, NH-CO-phenyl;
R6 and R7 are, independently of one another, H, (C₁-C₆)-alkyl, (C₁-C₆)-alkyl-O-(C₁-C₆)-alkyl, O-(C₁-C₆)-alkyl, (C₃-C₆)-cycloalkyl, CO-(C₁-C₆)-alkyl, (C₁-C₆)-alkyl-NH-C(O)-(C₁-C₆)-alkyl, (C₁-C₆)-alkyl-NH-(C₁-C₆)-alkyl, (C₁-C₆)-alkyl-N-[(C₁-C₆)-alkyl]₂, (C₁-C₆)-alkyl-O-phenyl, CHO, CO-phenyl, (CH₂)ₙ-Ar, where n can be 0 - 6, and Ar can be equal to phenyl, biphenylyl, 1- or 2-naphthyl, 1- or 2-tetrahydrofuranyl, 2-, 3- or 4-pyridyl, 2- or 3-thienyl, 2- or 3-furyl, 2-, 4- or 5-thiazolyl, 2-, 4- or 5-oxazolyl, 1-pyrazolyl, 3-, 4- or 5-isoxazolyl, (C₃-C₆)-cycloalkyl, piperidinyl, pyrrolidinyl, 2- or 3-pyrrolyl, 2- or 3-pyridazinyl, 2-, 4- or 5-pyrimidinyl, 2-pyrazinyl, 2-(1,3,5-triazinyl), 2-, 3- or 4-morpholinyl, 2- or 5-benzimidazolyl, 2-benzothiazolyl, 1,2,4-triazol-3-yl, 1,2,4-triazol-5-yl, tetrazol-5-yl, indol-3-yl, indol-5-yl or N-methyl-imidazol-2-, -4- or -5-yl, and Ar may be substituted up to twice by F, Cl, Br, OH, CF₃, NO₂, CN, OCF₃, O-CH₂-O, O-(C₁-C₆)-alkyl, S-(C₁-C₆)-alkyl, SO-(C₁-C₆)-alkyl, SO₂-(C₁-C₆)-alkyl, (C₁-C₆)-alkyl, (C₃-C₆)-cycloalkyl, COOH, COO(C₁-C₆)alkyl, COO(C₃-C₆)cycloalkyl, CONH₂, CONH(C₁-C₆)alkyl, CON[(C₁-C₆)alkyl]₂, CONH(C₃-C₆)cycloalkyl, NH₂, NH-CO-(C₁-C₆)-alkyl, NH-CO-phenyl, pyrrolidin-1-yl, morpholin-1-yl, piperidin-1-yl, piperazin-1-yl, 4-methyl-piperazin-1-yl, (CH₂)ₙ-phenyl, O-(CH₂)ₙ-phenyl, S-(CH₂)ₙ-phenyl, SO₂-(CH₂)ₙ-phenyl, where n = 0-3;
and of their physiologically tolerated salts and physiologically functional derivatives for producing a medicine for the prevention and treatment of hyperlipidemia.

2. The use of compounds of formula I as claimed in claim 1, wherein the meanings are
R1 NR6R7, pyrrolidine, piperidine, piperazine, tetrahydropyridine, it being possible for each of the rings to be substituted by phenyl, (C₁-C₆)-alkyl-phenyl, (C₁-C₆)-alkyl, (C₁-C₆)-alkyl-OH, O-phenyl, S-phenyl, (CO)-(C₁-C₆)-alkyl, (CO)-phenyl, where the phenyl substituent is unsubstituted or up to disubstituted by F, Cl, Br, CF₃, CN, OCF₃, O-(C₁-C₆)-alkyl, S-(C₁-C₆)-alkyl, (C₁-C₆)-alkyl, (C₃-C₆)-cycloalkyl, COOH, COO(C₁-C₆)-alkyl, COO(C₃-C₆)cycloalkyl, CONH₂, CONH(C₁-C₆)alkyl, CON[(C₁-C₆)alkyl]₂, NH₂, NH-CO-(C₁-C₆)-alkyl, NH-CO-phenyl;
R6, R7 independently of one another H, (C₁-C₆)-alkyl, (C₁-C₆)-alkyl-O-(C₁-C₆)-alkyl, (C₃-C₆)-cycloalkyl, CO-(C₁-C₆)-alkyl, (C₁-C₆)-alkyl-NH-C(O)-(C₁-C₆)-alkyl, (C₁-C₆)-alkyl-NH-(C₁-C₆)-alkyl, (C₁-C₆)-alkyl-N-[(C₁-C₆)-alkyl]₂,
(CH₂)ₙ-Ar, where n can be 0 - 6, and Ar can be equal to phenyl, biphenylyl, 1- or 2-naphthyl, 2-, 3- or 4-pyridyl, 2- or 3-thienyl, 2-, 4- or 5-thiazolyl, 2-, 4- or 5-oxazolyl, 3- or 5-isoxazolyl, (C₃-C₆)-cycloalkyl, piperidinyl, pyrrolidinyl, 2-, 4- or 5-pyrimidinyl, 2-, 3- or 4-morpholinyl, 2- or 5-benzimidazolyl, 2-benzothiazolyl or indol-3-yl, indol-5-yl, and Ar may be up to disubstituted by F, Cl, Br, OH, CF₃, NO₂, CN, OCF₃, O-(C₁-C₆)-alkyl, S-(C₁-C₆)-alkyl, SO-(C₁-C₆)-alkyl, SO₂-(C₁-C₆)-alkyl, (C₁-C₆)-alkyl, (C₃-C₆)-cycloalkyl, COOH, COO(C₁-C₆)alkyl, COO(C₃-C₆)cycloalkyl, CONH₂, CONH(C₁-C₆)alkyl, NH₂, NH-CO-phenyl, (CH₂)ₙ-phenyl, O-(CH₂)ₙ-phenyl, S-(CH₂)ₙ-phenyl, where n = 0-3;
R2 NR8R9, piperazine, it being possible for piperazine to be substituted by (C₁-C₆)-alkyl-phenyl, (C₁-C₆)-alkyl, (C₁-C₆)-alkyl-OH, O-phenyl, S-phenyl, (CO)-(C₁-C₆)-alkyl, (CO)-phenyl;
R8, R9 independently of one another H, (C₁-C₆)-alkyl, (C₃-C₆)-cycloalkyl, CO-(C₁-C₆)-alkyl, (C₁-C₆)-alkyl-NH-(C₁-C₆)-alkyl, (C₁-C₆)-alkyl-N-[(C₁-C₆)-alkyl]₂,
(CH₂)ₙ-Ar, where n can be 0 - 6, and Ar can be equal to phenyl, 2-, 3- or 4-pyridyl, piperidinyl, pyrrolidinyl, morpholinyl;
X NR10R11, pyrrolidine, piperidine, piperazine, morpholine, it being possible for each of the rings to be substituted by phenyl, (C₁-C₆)alkyl-phenyl, (C₁-C₆)-alkyl, (C₁-C₆)-alkyl-OH, O-phenyl, S-phenyl, (CO)-(C₁-C₆)-alkyl, (CO)-phenyl, where the phenyl substituent is unsubstituted or up to disubstituted by F, Cl, Br, CF₃, CN, OCF₃, O-(C₁-C₆)-alkyl, (C₁-C₆)-alkyl, (C₃-C₆)-cycloalkyl, COOH, COO(C₁-C₆)-alkyl, COO(C₃-C₆)cycloalkyl, CONH₂, CONH(C₁-C₆)alkyl, CON[(C₁-C₆)alkyl]₂, NH₂, NH-CO-(C₁-C₆)-alkyl, NH-CO-phenyl;
R10, R11 independently of one another H, (C₁-C₆)-alkyl, (C₁-C₆)-alkyl-O-(C₁-C₆)-alkyl, (C₃-C₆)-cycloalkyl, CO-(C₁-C₆)-alkyl, (C₁-C₆)-alkyl-NH-(C₁-C₆)-alkyl, (C₁-C₆)-alkyl-N-[(C₁-C₆)-alkyl]₂, CO-phenyl, (CH₂)ₙ-Ar, where n can be 0 - 6, and Ar can be equal to phenyl, biphenyl, 1- or 2-naphthyl, 1- or 2-tetrahydrofuranyl, 2-, 3- or 4-pyridyl, 2- or 3-thienyl, 2- or 3-furyl, 2-, 4- or 5-thiazolyl, 2-, 4- or 5-oxazolyl, 3- or 5-isoxazolyl, piperidinyl, pyrrolidinyl, 2-, 4- or 5-pyrimidinyl, 2-, 3- or 4-morpholinyl, 2-benzothiazolyl, and Ar may be disubstituted by F, Cl, Br, OH, CF₃, NO₂, CN, OCF₃, O-(C₁-C₆)-alkyl, S-(C₁-C₆)-alkyl, SO-(C₁-C₆)-alkyl, SO₂-(C₁-C₆)-alkyl, (C₁-C₆)-alkyl, (C₃-C₆)-cycloalkyl, CONH₂, CONH(C₁-C₆)alkyl, CON[(C₁-C₆)alkyl]₂, NH-CO-(C₁-C₆)-alkyl, NH-CO-phenyl, (CH₂)ₙ-phenyl, where n = 0-3;
and of their physiologically tolerated salts and physiologically functional derivatives for producing a medicine for the prevention and treatment of hyperlipidemia.

3. The use of compounds of formula I as claimed in claim 1 or 2, wherein the meanings are
R1 NR6R7, piperidine, piperazine, tetrahydropyridine, it being possible for each of the rings to be substituted by phenyl, (C₁-C₆)-alkyl-phenyl;
R6, R7 independently of one another H, (C₁-C₆)-alkyl, (C₁-C₆)-alkyl-O-(C₁-C₆)-alkyl, (C₃-C₆)-cycloalkyl, (C₁-C₆)-alkyl-NH-C(O)-(C₁-C₆)-alkyl, (C₁-C₆)-alkyl-NH-(C₁-C₆)-alkyl, (C₁-C₆)-alkyl-N-[(C₁-C₆)-alkyl]₂,
(CH₂)ₙ-Ar, where n can be 0 - 6, and Ar can be equal to phenyl, 1- or 2-naphthyl, 2-, 3- or 4-pyridyl, (C₃-C₆)-cycloalkyl, piperidinyl, pyrrolidinyl, 2-, 4- or 5-pyrimidinyl, 2-, 3- or 4-morpholinyl, and Ar may be up to disubstituted by F, Cl, Br, OH, CF₃, NO₂, CN, OCF₃, O-(C₁-C₆)-alkyl, (C₁-C₆)-alkyl, NH₂;
R2 NR8R9, piperazine, it being possible for piperazine to be substituted by (C₁-C₆)-alkyl;
R8, R9 independently of one another H, (C₁-C₆)-alkyl, (C₃-C₆)-cycloalkyl, CO-(C₁-C₆)-alkyl, (C₁-C₆)-alkyl-NH-(C₁-C₆)-alkyl, (C₁-C₆)-alkyl-N-[(C₁-C₆)-alkyl]₂,
(CH₂)ₙ-Ar, where n can be 0 - 6, and Ar can be equal to phenyl, 2-, 3- or 4-pyridyl, piperidinyl, pyrrolidinyl, morpholinyl;
X NR10R11, pyrrolidine, piperidine, morpholine, it being possible for each of the rings to be substituted by phenyl, (C₁-C₆)-alkyl-phenyl;
R10, R11 independently of one another H, (C₁-C₆)-alkyl, (C₁-C₆)-alkyl-O-(C₁-C₆)-alkyl, (C₃-C₆)-cycloalkyl, (C₁-C₆)-alkyl-NH-(C₁-C₆)-alkyl, (C₁-C₆)-alkyl-N-[(C₁-C₆)-alkyl]₂,
(CH₂)ₙ-Ar, where n can be 0 - 6, and Ar equals phenyl, 2- or 3-thienyl;
and of their physiologically tolerated salts for producing a medicine for the prevention and treatment of hyperlipidemia.

4. The use of the compounds as claimed in one or more of claims 1 to 3 in combination with one or more lipid-lowering agents for producing a medicine for the prophylaxis or treatment of hyperlipidemia.

5. The use of compounds of formula I 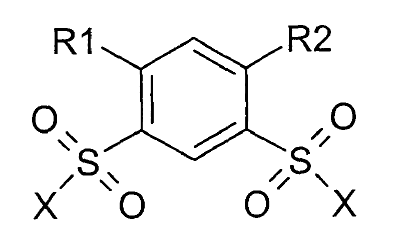 in which
X, R1, R2 are, independently of one another, NR6R7, pyrrolidine, piperidine, piperazine, morpholine, tetrahydropyridine, it being possible for each of the rings to be substituted by phenyl, (C₁-C₆)-alkyl-phenyl, (C₁-C₆)-alkyl, (C₁-C₆)-alkyl-OH, O-phenyl, S-phenyl, (CO)-(C₁-C₆)-alkyl, (CO)-phenyl, where the phenyl substituent is unsubstituted or up to disubstituted by F, Cl, Br, OH, CF₃, CN, OCF₃, O-(C₁-C₆)-alkyl, S-(C₁-C₆)-alkyl, SO-(C₁-C₆)-alkyl, SO₂-(C₁-C₆)-alkyl, (C₁-C₆)-alkyl, (C₃-C₆)-cycloalkyl, COOH, COO(C₁-C₆)alkyl, COO(C₃-C₆)cycloalkyl, CONH₂, CONH(C₁-C₆)alkyl, CON[(C₁-C₆)alkyl]₂, CONH(C₃-C₆)cycloalkyl, NH₂, NH-CO-(C₁-C₆)-alkyl, NH-CO-phenyl;
R6 and R7 are, independently of one another, H, (C₁-C₆)-alkyl, (C₁-C₆)-alkyl-O-(C₁-C₆)-alkyl, O-(C₁-C₆)-alkyl, (C₃-C₆)-cycloalkyl, CO-(C₁-C₆)-alkyl, (C₁-C₆)-alkyl-NH-C(O)-(C₁-C₆)-alkyl, (C₁-C₆)-alkyl-NH-(C₁-C₆)-alkyl, (C₁-C₆)-alkyl-N-[(C₁-C₆)-alkyl]₂, (C₁-C₆)-alkyl-O-phenyl, CHO, CO-phenyl,
(CH₂)ₙ-Ar, where n can be 0 - 6, and Ar can be equal to phenyl, biphenylyl, 1- or 2-naphthyl, 1- or 2-tetrahydrofuranyl, 2-, 3- or 4-pyridyl, 2- or 3-thienyl, 2- or 3-furyl, 2-, 4- or 5-thiazolyl, 2-, 4- or 5-oxazolyl, 1-pyrazolyl, 3-, 4- or 5-isoxazolyl, (C₃-C₆)-cycloalkyl, piperidinyl, pyrrolidinyl, 2- or 3-pyrrolyl, 2- or 3-pyridazinyl, 2-, 4- or 5-pyrimidinyl, 2-pyrazinyl, 2-(1,3,5-triazinyl), 2-, 3- or 4-morpholinyl, 2- or 5-benzimidazolyl, 2-benzothiazolyl, 1,2,4-triazol-3-yl, 1,2,4-triazol-5-yl, tetrazol-5-yl, indol-3-yl, indol-5-yl or N-methyl-imidazol-2-, -4- or -5-yl, and Ar may be substituted up to twice by F, Cl, Br, OH, CF₃, NO₂, CN, OCF₃, O-CH₂-O, O-(C₁-C₆)-alkyl, S-(C₁-C₆)-alkyl, SO-(C₁-C₆)-alkyl, SO₂-(C₁-C₆)-alkyl, (C₁-C₆)-alkyl, (C₃-C₆)-cycloalkyl, COOH, COO(C₁-C₆)alkyl, COO(C₃-C₆)cycloalkyl, CONH₂, CONH(C₁-C₆)alkyl, CON[(C₁-C₆)alkyl]₂, CONH(C₃-C₆)cycloalkyl, NH₂, NH-CO-(C₁-C₆)-alkyl, NH-CO-phenyl, pyrrolidin-1-yl, morpholin-1-yl, piperidin-1-yl, piperazin-1-yl, 4-methylpiperazin-1-yl, (CH₂)ₙ-phenyl, O-(CH₂)ₙ-phenyl, S-(CH₂)ₙ-phenyl, SO₂-(CH₂)ₙ-phenyl, where n = 0-3;
and of their physiologically tolerated salts and physiologically functional derivatives for producing a medicine for the prevention and treatment of arteriosclerosis.

6. The use of compounds of formula I as claimed in claim 5, wherein the meanings are
R1 NR6R7, pyrrolidine, piperidine, piperazine, tetrahydropyridine, it being possible for each of the rings to be substituted by phenyl, (C₁-C₆)-alkyl-phenyl, (C₁-C₆)-alkyl, (C₁-C₆)-alkyl-OH, O-phenyl, S-phenyl, (CO)-(C₁-C₆)-alkyl, (CO)-phenyl, where the phenyl substituent is unsubstituted or up to disubstituted by F, Cl, Br, CF₃, CN, OCF₃, O-(C₁-C₆)-alkyl, S-(C₁-C₆)-alkyl, (C₁-C₆)-alkyl, (C₃-C₆)-cycloalkyl, COOH, COO(C₁-C₆)-alkyl, COO(C₃-C₆)cycloalkyl, CONH₂, CONH(C₁-C₆)alkyl, CON[(C₁-C₆)alkyl]₂, NH₂, NH-CO-(C₁-C₆)-alkyl, NH-CO-phenyl;
R6, R7 independently of one another H, (C₁-C₆)-alkyl, (C₁-C₆)-alkyl-O-(C₁-C₆)-alkyl, (C₃-C₆)-cycloalkyl, CO-(C₁-C₆)-alkyl, (C₁-C₆)-alkyl-NH-C(O)-(C₁-C₆)-alkyl, (C₁-C₆)-alkyl-NH-(C₁-C₆)-alkyl, (C₁-C₆)-alkyl-N-[(C₁-C₆)-alkyl]₂,
(CH₂)ₙ-Ar, where n can be 0 - 6, and Ar can be equal to phenyl, biphenylyl, 1- or 2-naphthyl, 2-, 3- or 4-pyridyl, 2- or 3-thienyl, 2-, 4- or 5-thiazolyl, 2-, 4- or 5-oxazolyl, 3- or 5-isoxazolyl, (C₃-C₆)-cycloalkyl, piperidinyl, pyrrolidinyl, 2-, 4- or 5-pyrimidinyl, 2-, 3- or 4-morpholinyl, 2- or 5-benzimidazolyl, 2-benzothiazolyl or indol-3-yl, indol-5-yl, and Ar may be up to disubstituted by F, Cl, Br, OH, CF₃, NO₂, CN, OCF₃, O-(C₁-C₆)-alkyl, S-(C₁-C₆)-alkyl, SO-(C₁-C₆)-alkyl, SO₂-(C₁-C₆)-alkyl, (C₁-C₆)-alkyl, (C₃-C₆)-cycloalkyl, COOH, COO(C₁-C₆)alkyl, COO(C₃-C₆)cycloalkyl, CONH₂, CONH(C₁-C₆)alkyl, NH₂, NH-CO-phenyl, (CH₂)ₙ-phenyl, O-(CH₂)ₙ-phenyl, S-(CH₂)ₙ-phenyl, where n = 0-3;
R2 NR8R9, piperazine, it being possible for piperazine to be substituted by (C₁-C₆)-alkyl-phenyl, (C₁-C₆)-alkyl, (C₁-C₆)-alkyl-OH, O-phenyl, S-phenyl, (CO)-(C₁-C₆)-alkyl, (CO)-phenyl;
R8, R9 independently of one another H, (C₁-C₆)-alkyl, (C₃-C₆)-cycloalkyl, CO-(C₁-C₆)-alkyl, (C₁-C₆)-alkyl-NH-(C₁-C₆)-alkyl, (C₁-C₆)-alkyl-N-[(C₁-C₆)-alkyl]_{2,}
(CH₂)ₙ-Ar, where n can be 0 - 6, and Ar can be equal to phenyl, 2-, 3- or 4-pyridyl, piperidinyl, pyrrolidinyl, morpholinyl;
X NR10R11, pyrrolidine, piperidine, piperazine, morpholine, it being possible for each of the rings to be substituted by phenyl, (C₁-C₆)alkyl-phenyl, (C₁-C₆)-alkyl, (C₁-C₆)-alkyl-OH, O-phenyl, S-phenyl, (CO)-(C₁-C₆)-alkyl, (CO)-phenyl, where the phenyl substituent is unsubstituted or up to disubstituted by F, Cl, Br, CF₃, CN, OCF₃, O-(C₁-C₆)-alkyl, (C₁-C₆)-alkyl, (C₃-C₆)-cycloalkyl, COOH, COO(C₁-C₆)-alkyl, COO(C₃-C₆)cycloalkyl, CONH₂, CONH(C₁-C₆)alkyl, CON[(C₁-C₆)alkyl]₂, NH₂, NH-CO-(C₁-C₆)-alkyl, NH-CO-phenyl;
R10, R11 independently of one another H, (C₁-C₆)-alkyl, (C₁-C₆)-alkyl-O-(C₁-C₆)-alkyl, (C₃-C₆)-cycloalkyl, CO-(C₁-C₆)-alkyl, (C₁-C₆)-alkyl-NH-(C₁-C₆)-alkyl, (C₁-C₆)-alkyl-N-[(C₁-C₆)-alkyl]₂, CO-phenyl, (CH₂)ₙ-Ar, where n can be 0 - 6, and Ar can be equal to phenyl, biphenylyl, 1- or 2-naphthyl, 1- or 2-tetrahydrofuranyl, 2-, 3- or 4-pyridyl, 2- or 3-thienyl, 2- or 3-furyl, 2-, 4- or 5-thiazolyl, 2-, 4- or 5-oxazolyl, 3- or 5-isoxazolyl, piperidinyl, pyrrolidinyl, 2-, 4- or 5-pyrimidinyl, 2-, 3- or 4-morpholinyl, 2-benzothiazolyl, and Ar may be disubstituted by F, Cl, Br, OH, CF₃, NO₂, CN, OCF₃, O-(C₁-C₆)-alkyl, S-(C₁-C₆)-alkyl, SO-(C₁-C₆)-alkyl, SO₂-(C₁-C₆)-alkyl, (C₁-C₆)-alkyl, (C₃-C₆)-cycloalkyl, CONH₂, CONH(C₁-C₆)alkyl, CON[(C₁-C₆)alkyl]₂, NH-CO-(C₁-C₆)-alkyl, NH-CO-phenyl, (CH₂)ₙ-phenyl, where n = 0-3;
and of their physiologically tolerated salts and physiologically functional derivatives for producing a medicine for the prevention and treatment of arteriosclerosis.

7. The use of compounds of formula I as claimed in claim 5 or 6, wherein the meanings are
R1 NR6R7, piperidine, piperazine, tetrahydropyridine, it being possible for each of the rings to be substituted by phenyl, (C₁-C₆)-alkyl-phenyl;
R6, R7 independently of one another H, (C₁-C₆)-alkyl, (C₁-C₆)-alkyl-O-(C₁-C₆)-alkyl, (C₃-C₆)-cycloalkyl, (C₁-C₆)-alkyl-NH-C(O)-(C₁-C₆)-alkyl, (C₁-C₆)-alkyl-NH-(C₁-C₆)-alkyl, (C₁-C₆)-alkyl-N-[(C₁-C₆)-alkyl]₂,
(CH₂)ₙ-Ar, where n can be 0 - 6, and Ar can be equal to phenyl, 1- or 2-naphthyl, 2-, 3- or 4-pyridyl, (C₃-C₆)-cycloalkyl, piperidinyl, pyrrolidinyl, 2-, 4- or 5-pyrimidinyl, 2-, 3- or 4-morpholinyl, and Ar may be up to disubstituted by F, Cl, Br, OH, CF₃, NO₂, CN, OCF₃, O-(C₁-C₆)-alkyl, (C₁-C₆)-alkyl, NH₂;
R2 NR8R9, piperazine, it being possible for piperazine to be substituted by (C₁-C₆)-alkyl;
R8, R9 independently of one another H, (C₁-C₆)-alkyl, (C₃-C₆)-cycloalkyl, CO-(C₁-C₆)-alkyl, (C₁-C₆)-alkyl-NH-(C₁-C₆)-alkyl, (C₁-C₆)-alkyl-N-[(C₁-C₆)-alkyl]₂,
(CH₂)ₙ-Ar, where n can be 0 - 6, and Ar can be equal to phenyl, 2-, 3- or 4-pyridyl, piperidinyl, pyrrolidinyl, morpholinyl;
X NR10R11, pyrrolidine, piperidine, morpholine, it being possible for each of the rings to be substituted by phenyl, (C₁-C₆)-alkylphenyl;
R10, R11 independently of one another H, (C₁-C₆)-alkyl, (C₁-C₆)-alkyl-O-(C₁-C₆)-alkyl, (C₃-C₆)-cycloalkyl, (C₁-C₆)-alkyl-NH-(C₁-C₆)-alkyl, (C₁-C₆)-alkyl-N-[(C₁-C₆)-alkyl]₂,
(CH₂)ₙ-Ar, where n can be 0 - 6, and Ar equals phenyl, 2- or 3-thienyl;
and of their physiologically tolerated salts for producing a medicine for the prevention and treatment of arteriosclerosis.

8. The use of the compounds as claimed in one or more of claims 5 to 7 in combination with one or more lipid-lowering agents for producing a medicine for the prophylaxis or treatment of arteriosclerosis.

9. The use of the compounds as claimed in one or more of claims 1 to 3 in combination with one or more lipid-lowering agents for producing a medicine for the prophylaxis or treatment of hyperlipidemia.

## Revendications

1. Utilisation des composés de formule I, 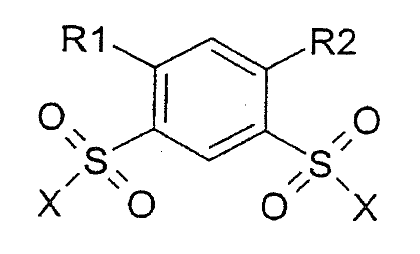 dans laquelle
X, R1, R2 représentent chacun indépendamment NR6R7, un cycle pyrrolidine, pipéridine, pipérazine, morpholine, tétrahydropyridine, les cycles pouvant être substitués chacun par phényle, alkyl(C₁-C₆)-phényle, alkyle en C₁-C₆, alkyl(C₁-C₆)-OH, O-phényle, S-phényle, (CO)-alkyle(C₁-C₆), (CO)-phényle, le substituant phényle étant non substitué ou jusqu'à deux fois substitué par F, Cl, Br, OH, CF₃, CN, OCF₃, O-alkyle(C₁-C₆), S-alkyle(C₁-C₆), SO-alkyle(C₁-C₆), SO₂-alkyle(C₁-C₆), alkyle en C₁-C₆, cycloalkyle en C₃-C₆, COOH, COO-alkyle(C₁-C₆), COO-cycloalkyle(C₃-C₆), CONH₂, CONH-alkyle(C₁-C₆), CON[alkyle(C₁-C₆)]₂, CONH-cycloalkyle(C₃-C₆), NH₂, NH-CO-alkyle(C₁-C₆), NH-CO-phényle ;
R6 et R7 représentent, indépendamment l'un de l'autre, H, un groupe alkyle en C₁-C₆, alkyl(C₁-C₆)-O-alkyle(C₁-C₆), O-alkyle en C₁-C₆, cycloalkyle en C₃-C₆, CO-alkyle(C₁-C₆), alkyl(C₁-C₆)-NH-C(O)-alkyle(C₁-C₆), alkyl(C₁-C₆)-NH-alkyle(C₁-C₆), alkyl (C₁-C₆)-N-[alkyle(C₁-C₆)]₂, alkyl(C₁-C₆)-O-phényle, CHO, CO-phényle, (CH₂)ₙ-Ar, n pouvant être égal à 0-6 et Ar pouvant être un radical phényle, biphénylyle, 1- ou 2-naphtyle, 1- ou 2-tétrahydrofurannyle, 2-, 3- ou 4-pyridyle, 2- ou 3-thiényle, 2- ou 3-furyle, 2-, 4- ou 5-thiazolyle, 2-, 4- ou 5-oxazolyle, 1-pyrazolyle, 3-, 4- ou 5-isoxazolyle, cycloalkyle en C₃-C₆, pipéridinyle, pyrrolidinyle, 2- ou 3-pyrrolyle, 2- ou 3-pyridazinyle, 2-, 4- ou 5-pyrimidinyle, 2-pyrazinyle, 2-(1,3,5-triazinyle), 2-, 3- ou 4-morpholinyle, 2- ou 5-benzimidazolyle, 2-benzothiazolyle, 1,2,4-triazol-3-yle, 1,2,4-triazol-5-yle, tétrazol-5-yle, indol-3-yle, indol-5-yle ou N-méthyl-imidazol-2-, -4- ou -5-yle et Ar pouvant être jusqu'à deux fois substitué par F, Cl, Br, OH, CF₃, NO₂, CN, OCF₃, O-CH₂-O, O-alkyle(C₁-C₆), S-alkyle(C₁-C₆), SO-alkyle(C₁-C₆), SO₂-alkyle(C₁-C₆), alkyle en C₁-C₆, cycloalkyle en C₃-C₆, COOH, COO-alkyle(C₁-C₆), COO-cycloalkyle(C₃-C₆), CONH₂, CONH-alkyle(C₁-C₆), CON[alkyle(C₁-C₆)]₂, CONH-cycloalkyle(C₃-C₆), NH₂, NH-CO-alkyle(C₁-C₆), NH-CO-phényle, pyrrolidin-1-yle, morpholin-1-yle, pipéridin-1-yle, pipérazin-1-yle, 4-méthyl-pipérazin-1-yle, (CH₂)ₙ-phényle, O-(CH₂)ₙ-phényle, S-(CH2)ₙ-phényle, SO₂-(CH₂)ₙ-phényle, n étant égal à 0-3 ;
ainsi que de leurs sels physiologiquement acceptables et dérivés physiologiquement fonctionnels, pour la fabrication d'un médicament destiné à la prévention et au traitement de l'hyperlipidémie.

2. Utilisation des composés de formule I, selon la revendication 1, **caractérisée en ce que** dans ces composés
R1 représente NR6R7, un cycle pyrrolidine, pipéridine, pipérazine, tétrahydro-pyridine, les cycles pouvant être substitués chacun par phényle, alkyl(C₁-C₆)-phényle, alkyle en C₁-C₆, alkyl(C₁-C₆)-OH, O-phényle, S-phényle, (CO)-alkyle(C₁-C₆), (CO)-phényle, le substituant phényle étant non substitué ou jusqu'à deux fois substitué par F, Cl, Br, CF₃, CN, OCF₃, O-alkyle(C₁-C₆), S-alkyle(C₁-C₆), alkyle en C₁-C₆, cycloalkyle en C₃-C₆, COOH, COO-alkyle(C₁-C₆), COO-cycloalkyle(C₃-C₆), CONH₂, CONH-alkyle(C₁-C₆) CON[alkyle(C₁-C₆)]₂, NH₂, NH-CO-alkyle(C₁-C₆), NH-CO-phényle ;
R6 et R7 représentent, indépendamment l'un de l'autre, H, un groupe alkyle en C₁-C₆, alkyl(C₁-C₆)-O-alkyle(C₁-C₆), cycloalkyle en C₃-C₆, CO-alkyle(C₁-C₆), alkyl(C₁-C₆)-NH-C(O)-alkyle(C₁-C₆), alkyl(C₁-C₆)-NH-alkyle(C₁-C₆), alkyl (C₁-C₆)-N-[alkyle(C₁-C₆)]₂,
(CH₂)ₙ-Ar, n pouvant être égal à 0-6 et Ar pouvant être un radical phényle, biphénylyle, 1- ou 2-naphtyle, 2-, 3- ou 4-pyridyle, 2- ou 3-thiényle, 2-, 4- ou 5-thiazolyle, 2-, 4- ou 5-oxazolyle, 3- ou 5-isoxazolyle, cycloalkyle en C₃-C₆, pipéridinyle, pyrrolidinyle, 2-, 4- ou 5-pyrimidinyle, 2-, 3- ou 4-morpholinyle, 2- ou 5-benzimidazolyle, 2-benzothiazolyle ou indol-3-yle, indol-5-yle et Ar pouvant être jusqu'à deux fois substitué par F, Cl, Br, OH, CF₃, NO₂, CN, OCF₃, O-alkyle(C₁-C₆), S-alkyle(C₁-C₆), SO-alkyle(C₁-C₆), SO₂-alkyle(C₁-C₆), alkyle en C₁-C₆, cycloalkyle en C₃-C₆, COOH, COO-alkyle(C₁-C₆), COO-cycloalkyle(C₃-C₆), CONH₂, CONH-alkyle(C₁-C₆), NH₂, NH-CO-phényle, (CH₂)ₙ-phényle, O-(CH₂)ₙ-phényle, S-(CH₂₎ₙ-phényle, n étant égal à 0-3 ;
R2 représente NR8R9, un cycle pipérazine, le cycle pipérazine pouvant être substitué par alkyl(C₁-C₆)-phényle, alkyle en C₁-C₆, alkyl(C₁-C₆)-OH, O-phényle, S-phényle, (CO)-alkyle(C₁-C₆), (CO)-phényle ;
R8, R9 représentent, indépendamment l'un de l'autre, H, un groupe alkyle en C₁-C₆, cycloalkyle en C₃-C₆, CO-alkyle(C₁-C₆), alkyl(C₁₋C₆)-NH-alkyle(C₁-C₆), alkyl(C₁-C₆)-N-[alkyle(C₁-C₆)]₂,
(CH₂)ₙ-Ar, n pouvant être égal à 0-6 et Ar pouvant être un radical phényle, 2-, 3- ou 4-pyridyle, pipéridinyle, pyrrolidinyle, morpholinyle ;
X représente NR10R11, un cycle pyrrolidine, pipéridine, pipérazine, morpholine, les cycles pouvant être substitués chacun par phényle, alkyl(C₁-C₆)-phényle, alkyle en C₁-C₆, alkyl(C₁-C₆)-OH, O-phényle, S-phényle, (CO)-alkyle(C₁-C₆), (CO)-phényle, le substituant phényle étant non substitué ou jusqu'à deux fois substitué par F, Cl, Br, CF₃, CN, OCF₃, O-alkyle(C₁-C₆), alkyle en C₁-C₆, cycloalkyle en C₃-C₆, COOH, COO-alkyle(C₁-C₆), COO-cycloalkyle(C₃-C₆), CONH₂, CONH-alkyle(C₁-C₆), CON[alkyle(C₁-C₆)]₂, NH₂, NH-CO-alkyle(C₁-C₆). NH-CO-phényle ;
R10 et R11 représentent, indépendamment l'un de l'autre, H, un groupe alkyle en C₁-C₆, alkyl(C₁-C₆)-O-alkyle(C₁-C₆), cycloalkyle en C₃-C₆, CO-alkyle(C₁-C₆), alkyl(C₁-C₆)-NH-alkyle(C₁-C₆), alkyl(C₁-C₆)-N-[alkyle(C₁-C₆)]₂, CO-phényle, (CH₂)ₙ-Ar, n pouvant être égal à 0-6 et Ar pouvant être un radical phényle, biphényle, 1- ou 2-naphtyle, 1- ou 2-tétrahydropyrannyle, 2-, 3- ou 4-pyridyle, 2- ou 3-thiényle, 2- ou 3-furyle, 2-, 4- ou 5-thiazolyle, 2-, 4- ou 5-oxazolyle, 3- ou 5-isoxazolyle, pipéridinyle, pyrrolidinyle, 2-, 4- ou 5-pyrimidinyle, 2-, 3- ou 4-morpholinyle, 2-benzothiazolyle et Ar pouvant être jusqu'à deux fois substitué par F, Cl, Br, OH, CF₃, NO₂, CN, OCF₃, O-alkyle(C₁-C₆), S-alkyle(C₁-C₆), SO-alkyle(C₁-C₆), SO₂-alkyle(C₁-C₆), alkyle en C₁-C₆, cycloalkyle en C₃-C₆, CONH₂, CONH-alkyle(C₁-C₆), CON[alkyle(C₁-C₆)]₂, NH-CO-alkyle(C₁-C₆), NH-CO-phényle, (CH₂)ₙ-phényle, n étant égal à 0-3 ;
ainsi que de leurs sels physiologiquement acceptables et dérivés physiologiquement fonctionnels, pour la fabrication d'un médicament destiné à la prévention et au traitement de l'hyperlipidémie.

3. Utilisation des composés de formule I, selon la revendication 1 ou 2, **caractérisée en ce que** dans ces composés
R1 représente NR6R7, un cycle pipéridine, pipérazine, tétrahydropyridine, les cycles pouvant être substitués chacun par phényle, alkyl(C₁-C₆)-phényle ;
R6 et R7 représentent, indépendamment l'un de l'autre, H, un groupe alkyle en C₁-C₆, alkyl (C₁-C₆)-O-alkyle(C₁-C₆), cycloalkyle en C₃-C₆, alkyl(C₁-C₆)-NH-C(O)-alkyle(C₁-C₆) alkyl(C₁-C₆)-NH-alkyle(C₁-C₆), alkyl(C₁-C₆)-N-[alkyle(C₁-C₆)]₂,
(CH₂)ₙ-Ar, n pouvant être égal à 0-6 et Ar pouvant être un radical phényle, 1- ou 2-naphtyle, 2-, 3- ou 4-pyridyle, cycloalkyle en C₃-C₆, pipéridinyle, pyrrolidinyle, 2-, 4- ou 5-pyrimidinyle, 2-, 3- ou 4-morpholinyle et Ar pouvant être jusqu'à deux fois substitué par F, Cl, Br, OH, CF₃, NO₂, CN, OCF₃, O-alkyle(C₁-C₆), alkyle en C₁-C₆, NH₂ ;
R2 représente NR8R9, un cycle pipérazine, le cycle pipérazine pouvant être substitué par alkyle en C₁-C₆ ;
R8, R9 représentent, indépendamment l'un de l'autre, H, un groupe alkyle en C₁-C₆, cycloalkyle en C₃-C₆, CO-alkyle(C₁-C₆), alkyl(C₁-C₆)-NH-alkyle(C₁-C₆), alkyl(C₁-C₆)-N-[alkyle(C₁-C₆)]₂,
(CH₂)ₙ-Ar, n pouvant être égal à 0-6 et Ar pouvant être un radical phényle, 2-, 3- ou 4-pyridyle, pipéridinyle, pyrrolidinyle, morpholinyle ;
X représente NR10R11, un cycle pyrrolidine, pipéridine, morpholine, les cycles pouvant être substitués chacun par phényle, alkyl(C₁-C₆)-phényle ;
R10 et R11 représentent, indépendamment l'un de l'autre, H, un groupe alkyle en C₁-C₆, alkyl(C₁-C₆)-O-alkyle(C₁-C₆), cycloalkyle en C₃-C₆, alkyl(C₁-C₆)-NH-alkyle(C₁-C₆), alkyl(C₁-C₆)-N-[alkyle(C₁-C₆)]₂,
(CH₂)ₙ-Ar, n pouvant être égal à 0-6 et Ar pouvant être un radical phényle, 2- ou 3-thiényle ;
ainsi que de leurs sels physiologiquement acceptables, pour la fabrication d'un médicament destiné à la prévention et au traitement de l'hyperlipidémie.

4. Utilisation des composés selon une ou plusieurs des revendications 1 à 3, en association avec un ou plusieurs hypolipidémiants, pour la fabrication d'un médicament destiné à la prophylaxie ou au traitement de l'hyperlipidémie.

5. Utilisation des composés de formule I, 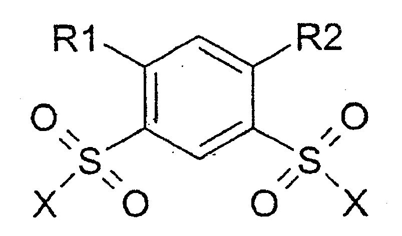 dans laquelle
X, R1, R2 représentent chacun indépendamment NR6R7, un cycle pyrrolidine, pipéridine, pipérazine, morpholine, tétrahydropyridine, les cycles pouvant être substitués chacun par phényle, alkyl(C₁-C₆)-phényle, alkyle en C₁-C₆, alkyl(C₁-C₆)-OH, O-phényle, S-phényle, (CO)-alkyle(C₁-C₆), (CO)-phényle, le substituant phényle étant non substitué ou jusqu'à deux fois substitué par F, Cl, Br, OH, CF₃, CN, OCF₃, O-alkyle(C₁-C₆), S-alkyle(C₁-C₆), SO-alkyle(C₁-C₆), SO₂-alkyle(C₁-C₆), alkyle en C₁-C₆, cycloalkyle en C₃-C₆, COOH, COO-alkyle(C₁-C₆). COO-cycloalkyle(C₃-C₆), CONH₂, CONH-alkyle(C₁-C₆), CON[alkyle(C₁-C₆)]₂, CONH-cycloalkyle(C₃-C₆), NH₂, NH-CO-alkyle(C₁-C₆), NH-CO-phényle ;
R6 et R7 représentent, indépendamment l'un de l'autre, H, un groupe alkyle en C₁-C₆, alkyl(C₁-C₆)-O-alkyle(C₁-C₆), O-alkyle en C₁-C₆, cycloalkyle en C₃-C₆, CO-alkyle(C₁-C₆), alkyl(C₁-C₆)-NH-C(O)-alkyle(C₁-C₆), alkyl(C₁-C₆)-NH-alkyle(C₁-C₆), alkyl (C₁-C₆)-N-[alkyle(C₁-C₆)]₂, alkyl(C₁-C₆)-O-phényle, CHO, CO-phényle,
(CH₂)ₙ-Ar, n pouvant être égal à 0-6 et Ar pouvant être un radical phényle, biphénylyle, 1- ou 2-naphtyle, 1- ou 2-tétrahydrofurannyle, 2-, 3- ou 4-pyridyle, 2- ou 3-thiényle, 2- ou 3-furyle, 2-, 4- ou 5-thiazolyle, 2-, 4- ou 5-oxazolyle, 1-pyrazolyle, 3-, 4- ou 5-isoxazolyle, cycloalkyle en C₃-C₆, pipéridinyle, pyrrolidinyle, 2- ou 3-pyrrolyle, 2- ou 3-pyridazinyle, 2-, 4- ou 5-pyrimidinyle, 2-pyrazinyle, 2-(1,3,5-triazinyle), 2-, 3- ou 4-morpholinyle, 2- ou 5-benzimidazolyle, 2-benzothiazolyle, 1,2,4-triazol-3-yle, 1,2,4-triazol-5-yle, tétrazol-5-yle, indol-3-yle, indol-5-yle ou N-méthyl-imidazol-2-, -4- ou -5-yle et Ar pouvant être jusqu'à deux fois substitué par F, Cl, Br, OH, CF₃, NO₂, CN, OCF₃, O-CH₂-O, O-alkyle(C₁-C₆), S-alkyle(C₁-C₆) SO-alkyle(C₁-C₆), SO₂-alkyle(C₁-C₆), alkyle en C₁-C₆, cycloalkyle en C₃-C₆, COOH, COO-alkyle(C₁-C₆), COO-cycloalkyle(C₃-C₆), CONH₂, CONH-alkyle(C₁-C₆), CON[alkyle(C₁-C₆)]₂, CONH-cycloalkyle(C₃-C₆), NH₂, NH-CO-alkyle(C₁-C₆), NH-CO-phényle, pyrrolidin-1-yle, morpholin-1-yle, pipéridin-1-yle, pipérazin-1-yle, 4-méthyl-pipérazin-1-yle, (CH₂)ₙ-phényle, O-(CH₂)ₙ-phényle, S-(CH₂)ₙ-phényle, SO₂-(CH₂)ₙ-phényle, n étant égal à 0-3 ;
ainsi que de leurs sels physiologiquement acceptables et dérivés physiologiquement fonctionnels, pour la fabrication d'un médicament destiné à la prévention et au traitement de l'artériosclérose.

6. Utilisation des composés de formule I, selon la revendication 5, **caractérisée en ce que** dans ces composés
R1 représente NR6R7, un cycle pyrrolidine, pipéridine, pipérazine, tétrahydro-pyridine, les cycles pouvant être substitués chacun par phényle, alkyl(C₁-C₆)-phényle, alkyle en C₁-C₆, alkyl(C₁-C₆)-OH, O-phényle, S-phényle, (CO)-alkyle(C₁-C₆), (CO)-phényle, le substituant phényle étant non substitué ou jusqu'à deux fois substitué par F, Cl, Br, CF₃, CN, OCF₃, O-alkyle(C₁-C₆), S-alkyle(C₁-C₆), alkyle en C₁-C₆, cycloalkyle en C₃-C₆, COOH, COO-alkyle(C₁-C₆) COO-cycloalkyle(C₃-C₆), CONH₂, CONH-alkyle(C₁-C₆), CON[alkyle(C₁-C₆)]₂, NH₂, NH-CO-alkyle(C₁-C₆), NH-CO-phényle ;
R6 et R7 représentent, indépendamment l'un de l'autre, H, un groupe alkyle en C₁-C₆, alkyl(C₁-C₆)-O-alkyle(C₁-C₆), cycloalkyle en C₃-C₆, CO-alkyle(C₁-C₆), alkyl(C₁-C₆)-NH-C(O)-alkyle(C₁-C₆), alkyl(C₁-C₆)-NH-alkyle(C₁-C₆), alkyl (C₁-C₆)-N-[alkyle(C₁-C₆)]₂,
(CH₂)ₙ-Ar, n pouvant être égal à 0-6 et Ar pouvant être un radical phényle, biphénylyle, 1- ou 2-naphtyle, 2-, 3- ou 4-pyridyle, 2- ou 3-thiényle, 2-, 4- ou 5-thiazolyle, 2-, 4- ou 5-oxazolyle, 3- ou 5-isoxazolyle, cycloalkyle en C₃-C₆, pipéridinyle, pyrrolidinyle, 2-, 4- ou 5-pyrimidinyle, 2-, 3- ou 4-morpholinyle, 2- ou 5-benzimidazolyle, 2-benzothiazolyle ou indol-3-yle, indol-5-yle et Ar pouvant être jusqu'à deux fois substitué par F, Cl, Br, OH, CF₃, NO₂, CN, OCF₃, O-alkyle(C₁-C₆), S-alkyle(C₁-C₆), SO-alkyle(C₁-C₆), SO₂-alkyle(C₁-C₆), alkyle en C₁-C₆, cycloalkyle en C₃-C₆, COOH, COO-alkyle(C₁-C₆), COO-cycloalkyle(C₃-C₆), CONH₂, CONH-alkyle(C₁-C₆), NH₂, NH-CO-phényle, (CH₂)ₙ-phényle, O-(CH₂)ₙ-phényle, S-(CH₂)ₙ-phényle, n étant égal à 0-3 ;
R2 représente NR8R9, un cycle pipérazine, le cycle pipérazine pouvant être substitué par alkyl(C₁-C₆)-phényle, alkyle en C₁-C₆, alkyl(C₁-C₆)-OH, O-phényle, S-phényle, (CO)-alkyle(C₁-C₆), (CO)-phényle ;
R8, R9 représentent, indépendamment l'un de l'autre, H, un groupe alkyle en C₁-C₆, cycloalkyle en C₃-C₆, CO-alkyle(C₁-C₆), alkyl(C₁-C₆)-NH-alkyle(C₁-C₆), alkyl(C₁-C₆) -N-[alkyle (C₁-C₆)]₂,
(CH₂)ₙ-Ar, n pouvant être égal à 0-6 et Ar pouvant être un radical phényle, 2-, 3- ou 4-pyridyle, pipéridinyle, pyrrolidinyle, morpholinyle ;
X représente NR10R11, un cycle pyrrolidine, pipéridine, pipérazine, morpholine, les cycles pouvant être substitués chacun par phényle, alkyl(C₁-C₆)-phényle, alkyle en C₁-C₆, alkyl(C₁-C₆)-OH, O-phényle, S-phényle, (CO)-alkyle(C₁-C₆), (CO)-phényle, le substituant phényle étant non substitué ou jusqu'à deux fois substitué par F, Cl, Br, OH, CF₃, CN, OCF₃, O-alkyle(C₁-C₆), alkyle en C₁-C₆, cycloalkyle en C₃-C₆, COOH, COO-alkyle(C₁-C₆), COO-cycloalkyle(C₃-C₆), CONH₂, CONH-alkyle(C₁-C₆), CON[alkyle (C₁-C₆)]₂, NH₂, NH-CO-alkyle(C₁-C₆), NH-CO-phényle ;
R10 et R11 représentent, indépendamment l'un de l'autre, H, un groupe alkyle en C₁-C₆, alkyl(C₁-C₆)-O-alkyle(C₁-C₆), cycloalkyle en C₃-C₆, CO-alkyle(C₁-C₆), alkyl(C₁-C₆)-NH-alkyle(C₁-C₆), alkyl(C₁-C₆)-N-[alkyle(C₁-C₆)]₂, CO-phényle, (CH₂)ₙ-Ar, n pouvant être égal à 0-6 et Ar pouvant être un radical phényle, biphénylyle, 1- ou 2-naphtyle, 1- ou 2-tétrahydropyrannyle, 2-, 3- ou 4-pyridyle, 2- ou 3-thiényle, 2- ou 3-furyle, 2-, 4- ou 5-thiazolyle, 2-, 4- ou 5-oxazolyle, 3- ou 5-isoxazolyle, pipéridinyle, pyrrolidinyle, 2-, 4- ou 5-pyrimidinyle, 2-, 3- ou 4-morpholinyle, 2-benzothiazolyle et Ar pouvant être jusqu'à deux fois substitué par F, Cl, Br, OH, CF₃, NO₂, CN, OCF₃, O-alkyle(C₁-C₆), S-alkyle(C₁-C₆), SO-alkyle(C₁-C₆), SO₂-alkyle(C₁-C₆), alkyle en C₁-C₆, cycloalkyle en C₃-C₆, CONH₂, CONH-alkyle(C₁-C₆), CON[alkyle(C₁-C₆)]₂, NH-CO-alkyle(C₁-C₆), NH-CO-phényle, (CH₂)ₙ-phényle, n étant égal à 0-3 ;
ainsi que de leurs sels physiologiquement acceptables et dérivés physiologiquement fonctionnels, pour la fabrication d'un médicament destiné à la prévention et au traitement de l'artériosclérose.

7. Utilisation des composés de formule I, selon la revendication 5 ou 6, **caractérisée en ce que** dans ces composés
R1 représente NR6R7, un cycle pipéridine, pipérazine, tétrahydropyridine, les cycles pouvant être substitués chacun par phényle, alkyl(C₁-C₆)-phényle ;
R6 et R7 représentent, indépendamment l'un de l'autre, H, un groupe alkyle en C₁-C₆, alkyl(C₁-C₆)-O-alkyle(C₁-C₆), cycloalkyle en C₃-C₆, alkyl(C₁-C₆)-NH-C(O)-alkyle(C₁-C₆), alkyl(C₁-C₆)-NH-alkyle(C₁-C₆), alkyl(C₁-C₆)-N-[alkyle(C₁-C₆)]₂,
(CH₂)ₙ-Ar, n pouvant être égal à 0-6 et Ar pouvant être un radical phényle, 1- ou 2-naphtyle, 2-, 3- ou 4-pyridyle, cycloalkyle en C₃-C₆, pipéridinyle, pyrrolidinyle, 2-, 4- ou 5-pyrimidinyle, 2-, 3- ou 4-morpholinyl, et Ar pouvant être jusqu'à deux fois substitué par F, Cl, Br, OH, CF₃, NO₂, CN, OCF₃, O-alkyle(C₁-C₆), alkyle en C₁-C₆, NH₂ ;
R2 représente NR8R9, un cycle pipérazine, le cycle pipérazine pouvant être substitué par alkyle en C₁-C₆ ;
R8, R9 représentent, indépendamment l'un de l'autre, H, un groupe alkyle en C₁-C₆, cycloalkyle en C₃-C₆, CO-alkyle(C₁-C₆), alkyl(C₁-C₆)-NH-alkyle(C₁-C₆), alkyl(C₁-C₆)-N-[alkyle(C₁-C₆)]₂,
(CH₂)ₙ-Ar, n pouvant être égal à 0-6 et Ar pouvant être un radical phényle, 2-, 3- ou 4-pyridyle, pipéridinyle, pyrrolidinyle, morpholinyle ;
X représente NR10R11, un cycle pyrrolidine, pipéridine, morpholine, les cycles pouvant être substitués chacun par phényle, alkyl(C₁-C₆)-phényle ;
R10 et R11 représentent, indépendamment l'un de l'autre, H, un groupe alkyle en C₁-C₆, alkyl(C₁-C₆)-O-alkyle(C₁-C₆), cycloalkyle en C₃-C₆, alkyl(C₁-C₆)-NH-alkyle(C₁-C₆), alkyl (C₁-C₆)-N-[alkyle(C₁-C₆)]₂,
(CH₂)ₙ-Ar, n pouvant être égal à 0-6 et Ar pouvant être un radical phényle, 2- ou 3-thiényle ;
ainsi que de leurs sels physiologiquement acceptables, pour la fabrication d'un médicament destiné à la prévention et au traitement de l'artériosclérose.

8. Utilisation des composés selon une ou plusieurs des revendications 5 à 7, en association avec un ou plusieurs hypolipidémiants, pour la fabrication d'un médicament destiné à la prophylaxie ou au traitement de l'artériosclérose.

9. Utilisation des composés selon une ou plusieurs des revendications 1 à 3, en association avec un ou plusieurs hypolipidémiants, pour la fabrication d'un médicament destiné à la prophylaxie ou au traitement de l'hyperlipidémie.
